(19) Europäisches Patentamt
European Patent Office
Office européen des brevets

(11) **EP 3 338 549 A1**

(12) **EUROPEAN PATENT APPLICATION**

(43) Date of publication:
**27.06.2018 Bulletin 2018/26**

(51) Int Cl.:
*A01N 25/02* (2006.01)     *A61K 9/14* (2006.01)
*A61L 24/04* (2006.01)     *A01N 1/02* (2006.01)
*C08B 37/02* (2006.01)     *C08J 3/075* (2006.01)
*C08J 3/24* (2006.01)     *C12N 5/00* (2006.01)

(21) Application number: **18152529.6**

(22) Date of filing: **20.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **20.11.2007   US 989176 P**

(62) Document number(s) of the earlier application(s) in accordance with Art. 76 EPC:
**08852924.3 / 2 222 159**

(71) Applicant: **Pioneer Surgical Orthobiologics, Inc.**
**Greenville, NC 27834 (US)**

(72) Inventors:
• **KLANN, Richard Chris**
  **Washington, NC 27889 (US)**
• **HILL, Ronald Stewart**
  **Greenville, NC 27834 (US)**
• **LAMBERTI, Francis V.**
  **Cary, NC 27511 (US)**

(74) Representative: **Gevers & Orès**
  **41 avenue de Friedland**
  **75008 Paris (FR)**

Remarks:
This application was filed on 19-01-2018 as a divisional application to the application mentioned under INID code 62.

(54) **CRYOPRESERVATION OF CELLS USING CROSS-LINKED BIOACTIVE HYDROGEL MATRIX PARTICLES**

(57) The present invention is directed to methods of cryopreserving cells and cryopreserved cells prepared according to the methods. In specific embodiments, the method comprises combining cells with a cross-linked hydrogel matrix in particulate form, the matrix comprising a polyglycan cross-linked to a polypeptide and subjecting the combination to cryopreservation conditions. In further embodiments, the invention provides cell-seeded compositions comprising cells and a cross-linked bioactive hydrogel matrix in particulate form, the matrix comprising a polyglycan cross-linked to a polypeptide, wherein the composition has been subjected to cryopreservation conditions. The cryopreserved cells can be thawed and used in methods of treatment without the need for intervening steps to make the cells viable for *in vivo* use.

EP 3 338 549 A1

**Description**

FIELD OF THE INVENTION

The present invention relates to cryopreservation of cells. More particularly, the invention relates to cryopreservation of cells on particles of a bioactive hydrogel matrix.

BACKGROUND OF THE INVENTION

Cryopreservation is a useful tool that enables stocks of cells to be stored and thus overcomes the need to have an ongoing store of all cell lines in culture at all times. Cryopreservation is particularly invaluable when dealing with cells of limited life span. Further advantages of cryopreservation include reduced risk of microbial contamination, reduced risk of cross contamination with other cell lines, reduced risk of genetic drift and morphological changes, and reduced costs (*e.g.,* consumables and staff time).

There has been much developmental work undertaken to ensure successful cryopreservation and resuscitation of a wide variety of cell lines of different cell types. The basic principle of successful cryopreservation has typically been based on a slow freeze and a quick thaw. Although the precise requirement may vary with different cell lines, it is generally recommended that cells should be cooled at a rate of around -1°C to -3°C per minute and thawed quickly by incubation in a 37°C waterbath for approximately 3-5 minutes. For successful cryopreservation of most cell lines, the following guidelines are further typically recommended: 1) cultures should be healthy with a viability of >90% and no signs of microbial contamination; 2) cultures should be in the log phase of growth (this can be achieved by using pre-confluent cultures *i.e.,* cultures that are below their maximum cell density and by changing the culture medium 24 hours before freezing); 3) a high concentration of serum/protein (>20%) should be used - in many cases serum is used at 90%; and 4) use a cryoprotectant such as dimethyl sulfoxide or glycerol to help protect the cells from rupture by the formation of ice crystals.

Cryopreservation of cells has become such a common aspect of laboratory techniques that the European Collection of Cell Cultures (ECACC), in cooperation with Sigma, has published handbook entitled "Fundamental Techniques in Cell Culture...a Laboratory Handbook," which provides details on standard cell cryopreservation techniques. Even when following recommended procedures, as described above, a common problem underlying cell cryopreservation is cell disruption between the initial culturing of the cells and the eventual use of the cells. A typical protocol for cryopreserving cells consists of the following steps: removing cells from culture (such as by trypsinizing to detach the cells from the substrate or using mechanical scraping); suspending in fresh medium; isolating cells for preservation; suspending cells in freeze medium; pipetting cell aliquots into ampules for freezing; placing ampules in -80 °C freezer; and transferring to liquid nitrogen storage vessel.

Cell disruption can particularly arise from the initial step of placing adherent cell cultures into suspension. Trypsinization, or other protease treatment, to detach adherent cells can be detrimental to cell integrity, particularly disturbing or destroying cell surface proteins. The detrimental effect of protease treatment is recognized in the art, and the ECACC Handbook, for example, recommends the use of cell scrapers to avoid removal of membrane markers/receptors of interest by enzymatic treatment. Mechanical removal of adherent cells, though, can also severely damage cultured cells, such as through cell membrane disruption or mechanical damage or removal of cell surface proteins.

The damaging effects associated with suspending adherent cultured cells must be addressed after thawing cryopreserved cells and before later use of the cells. Typically, such cell regeneration involves further culturing of the cells prior to final use. This is costly (both in time and resources) and overly complicates the entire cryopreservation process. Accordingly, it would be useful to have a cryopreservation process wherein cell disruption is avoided prior to the actual cryopreserving, thus obviating the need to re-culture resuscitated cells prior to use.

BRIEF SUMMARY OF THE INVENTION

The present invention provides a cryopreservation method that both simplifies and improves known methods. Moreover, the invention provides cryopreserved cells in a form allowing for immediate use after thawing. The invention particularly utilizes a stabilized cross-linked bioactive hydrogel matrix that provides a scaffold for cell attachment before, during, and after cryopreservation and that can be directly used *in vitro* or *in vivo* to deliver the previously cryopreserved cells to a site of interest or need. The bioactive hydrogel matrix can thus function as a growth substrate for cells, as a carrier for long-term storage during cryopreservation, and as a delivery device after resuscitation of cryopreserved cells. Moreover, the bioactive hydrogel matrix can actually provide therapeutic benefits in association with the cell delivery, including modulation of localized wound healing and tissue integration. The cells may be at least partially retained on an exposed surface on the hydrogel matrix particles. In further embodiments, the cells may be at least partially retained within one or more pores present in the hydrogel matrix particles.

A variety of hydrogels may be used according to the present invention. The hydrogel may be comprised completely of natural components, may be comprised completely of synthetic components, or may comprise a combination of natural and synthetic components. Examples of natural components include, but are not limited to, naturally occurring proteins and polypeptides and naturally occurring polyglycans, such as polysaccharides. Specific examples of synthetic hydrogels that may be used according to the invention include hydrogels comprising polyethylene glycol (PEG), acrylates, methacrylates (e.g., 2-hydroxyethyl methacrylate or pHEMA), and polyvinyl alcohol.

In certain embodiments, the hydrogel matrices of the invention may comprise a first high molecular weight component and a second high molecular weight component covalently cross-linked to the first high molecular weight component. The first high molecular weight component and the second high molecular weight component particularly can be selected from the group consisting of polyglycans and polypeptides. The polyglycan can particularly be a polysaccharide or a derivatized polysaccharide (e.g., sulfates, acetates, phosphates, and ammonium salts of polysaccharides) and can include polysaccharides such as commonly found in biofilms or extracellular matrices. The polyglycans, for example, and can be selected from the group consisting of dextran, heparan, heparin, hyaluronic acid, alginate, agarose, carageenan, amylopectin, amylose, glycogen, starch, cellulose, chitin, chitosan, heparan sulfate, chondroitin sulfate, dextran sulfate, dermatan sulfate, and keratan sulfate. The polypeptide can be selected from the group consisting of collagens, gelatins, keratin, decorin, aggrecan, glycoproteins, laminin, nidogen, fibulin, and fibrillin. In one embodiment, the polyglycan can be dextran and the polypeptide can be gelatin.

The matrix can further comprise one or more enhancing agents, such as polar amino acids, amino acid analogues, amino acid derivatives, and divalent cation chelators, such as ethylenediaminetetraacetic acid (EDTA) or salts thereof. In specific embodiments, the matrix can also comprise intact collagen. Preferred enhancing agents include polar amino acids, such as cysteine, arginine, lysine, and glutamic acid, EDTA or salts thereof, and mixtures or combinations thereof. Such enhancing agents are particularly useful when the hydrogel matrix comprises a polypeptide and a polyglycan.

The bioactive hydrogel matrix can be in particulate form when combined with the cells for cryopreservation. The hydrogel matrix can be formed into particles by various methods, such as lyophilization and milling the lyophilized matrix to a desired average particle size. In one embodiment, the particulate hydrogel matrix has an average particle size of about 0.01 mm to about 2 mm.

The hydrogel matrix particles can particularly be porous. In such embodiments, the particles may have an average pore size of about 100 $\mu$m to about 1000 $\mu$m. The particles may also have an average interconnectivity pore size of less than about 200 $\mu$m. In specific embodiments, the particles may have an average porosity of at least about 25%. The average porosity may alternately be in the range of about 30% to about 90%.

In one aspect, the present invention provides a method of cryopreserving cells. In a particular embodiment, the method comprises combining cells for cryopreservation with a cross-linked bioactive hydrogel matrix in particulate form. In specific embodiments, the hydrogel matrix can comprise a polyglycan cross-linked to a polypeptide. The combined cells and cross-linked bioactive hydrogel matrix are then subjected to cryopreservation conditions, which can include any conditions typically used in cryopreservation techniques. The cells for cryopreservation can be combined with the particulate cross-linked hydrogel matrix by any useful method, such as providing the cells in a suspension and adding the particulate cross-linked hydrogel matrix to the cell suspension, optionally with mixing. In another embodiment, the cells can be combined with the cross-linked hydrogel matrix by providing the cells for cryopreservation in a cell suspension and applying the cell suspension to the particulate cross-linked hydrogel matrix. In still another embodiment, the cells can be combined with the un-reacted components of the cross-linked hydrogel matrix and sprayed to form cross-linkable droplets containing cells in suspension.

In an embodiment, the invention is directed to a method of cryopreserving cells comprising subjecting to cryopreservation conditions particles of a cross-linked bioactive hydrogel matrix, said hydrogel matrix particles retaining cells for cryopreservation. The hydrogel matrix may comprise a polyglycan cross-linked to a polypeptide. The step of subjecting to cryopreservation conditions may comprise introducing the hydrogel matrix particles retaining the cells to an environment providing a cryopreservation temperature. Such cryopreservation temperature may particularly be a temperature sufficiently below 0 °C to slow or stop biological activity within the cells. For example, the cryopreservation temperature may be a temperature of less than about -20 °C. The step of subjecting to cryopreservation conditions can include contacting the hydrogel matrix particles retaining the cells with a cryoprotectant. In specific embodiments, the hydrogel matrix particles retaining the cells can be in a suspension, and the cryoprotectant can comprise a material effective for lowering the freezing temperature of the suspension, increasing the viscosity of the suspension, or both.

The hydrogel matrix particles can be suitable for retaining a specific number of cells, such as an average of at least about 20 cells. In some embodiments, the hydrogel matrix particles may each retain an average of about 10 cells to about 200 cells. The types of cells can vary. In some embodiments, the cells for cryopreservation can be selected from the group consisting of stem cells, progenitor cells, mesenchymal cells, pluripotent cells, multipotent cells, and combinations thereof. In specific embodiments, the cells for cryopreservation can be selected from the group consisting of mesenchymal stem cells, neural stem cells, muscle stem cells, adipose-derived adult stem (ADAS) cells, liver cells, pancreatic cells, chondrocytes, osteoblasts, adipocytes, fibroblasts, and combinations thereof.

In another aspect, the invention provides a cell-seeded composition. In specific embodiments, the composition can comprise cells and a cross-linked bioactive hydrogel matrix in particulate form.

In one embodiment, the invention is directed to a cell-seeded composition comprising particles of a cross-linked bioactive hydrogel matrix and cells that are at least partially retained by the hydrogel matrix particles. The hydrogel matrix may comprise a polyglycan cross-linked to a polypeptide. The composition can particularly be in a cryopreserved form.

In another embodiment, the invention is directed to a cell-seeded composition comprising particles of a cross-linked bioactive hydrogel matrix, cells that are at least partially retained by the hydrogel matrix particles, and a cryoprotectant. The hydrogel matrix may comprise a polyglycan cross-linked to a polypeptide.

In still another aspect, the present invention provides a method of administering viable cells to a site in a patient (i.e., administering cells *in vivo*). In one embodiment, the method comprises providing particles of a cross-linked bioactive hydrogel matrix and cells that are at least partially retained by the hydrogel matrix particles, the particles and the retained cells being in a cryopreserved form. The hydrogel matrix may comprise a polyglycan and a polypeptide. The method further may comprise thawing the cryopreserved particles and the retained cells, and administering the particles with the retained cells to the site in the patient. In further embodiments, the method may comprise, prior to the administering step, forming a suspension of the particles and the retained cells. The step of forming the suspension may particularly comprise combining the thawed particles and the retained cells with a thermoreversible hydrogel matrix, such as a hydrogel comprising a polyglycan and a polypeptide.

BRIEF DESCRIPTION OF THE DRAWINGS

Having thus described the invention in general terms, reference will now be made to the accompanying drawings, which are not necessarily drawn to scale, and wherein:

> FIG. 1 illustrates formation of open alpha chains derived from collagen monomers;
> FIG. 2A illustrates the effect of the association of the alpha chains with dextran;
> FIG. 2B illustrates the behavior of the alpha chains without association of the dextran;
> FIG. 3 illustrates the effect of other hydrogel matrix additives;
> FIG. 4 graphically illustrates cellular aggregation across various cell types in the presence of the bioactive hydrogel matrix of the present invention;
> FIG. 5 illustrates an embodiment of a covalently cross-linked gelatin/dextran matrix of the invention;
> FIG. 6 illustrates graphically the relationship of the overall strength of the cross-linked hydrogel matrix to the amount of dextran oxidation; and
> FIG. 7 illustrates the levels of ALP activity of culture medium over a 6-day culture period for SAOS cells seeded on cross-linked hydrogel matrix particles according to one embodiment of the invention, subjected to cryopreservation conditions, and thawed.

DETAILED DESCRIPTION OF THE INVENTION

The present invention now will be described more fully hereinafter with reference to the accompanying drawings, in which preferred embodiments of the invention are shown. These embodiments are provided so that this disclosure will be thorough and complete, and will fully convey the scope of the invention to those skilled in the art. Indeed, the invention may be embodied in many different forms and should not be construed as limited to the embodiments set forth herein; rather, these embodiments are provided so that this disclosure will satisfy applicable legal requirements. Like numbers refer to like elements throughout. As used in the specification, and in the appended claims, the singular forms "a", "an", "the", include plural referents unless the context clearly dictates otherwise.

The present invention provides a new method for working with cultured cells that allows for the preparation of cryopreserved cell populations that can be introduced *in vivo* for therapeutic uses without the need for *in vitro* culture or dissociation between resuscitating from the cryopreserved state and dispensing *in vivo*. This improved cell culture/cryopreservation method is made possible through provision of biocompatible material capable of functioning as a cell culture substrate, a cryopreservation substrate, and a biocompatible *in vivo* cell delivery device.

Biocompatibility is defined as the appropriate response of the host to a foreign material used for its intended application. Biocompatibility further refers to the interaction between the foreign material and the tissues and physiological systems of the patient treated with the foreign material. Protein binding and subsequent denaturation as well as cell adhesion and activation have been invoked as determinants of a material's biocompatibility. Biocompatibility also implies that the implant avoids detrimental effects from the host's various protective systems and remains functional for a significant period of time. *In vitro* tests designed to assess cytotoxicity or protein binding are routinely used for the measurement of a material's potential biocompatibility. In other words, the biocompatibility of a material is dependent upon its ability to be fully integrated with the surrounding tissue following implantation.

Previous research has shown that the specific interactions between cells and their surrounding extracellular matrix play an important role in the promotion and regulation of cellular repair and replacement processes (Hynes, S.O., "Integrins: a family of cell surface receptors" Cell 48:549-554 (1987)). Consequently, there has been a heightened interest in work related to biocompatible polymers useful in therapeutic applications. One particular class of polymers that have proven useful for such applications, including contact lens materials, artificial tendons, matrices for tissue engineering, and drug delivery systems, is hydrogels (Schacht, E., "Hydrogels prepared by crosslinking of gelatin with dextran dialdehyde" Reactive & Functional Polymers 33:109-116 (1997)). Hydrogels are commonly accepted to be materials consisting of a permanent, three-dimensional network of hydrophilic polymers with water filling the space between the polymer chains. Hydrogels may be obtained by copolymerizing suitable hydrophilic monomers, by chain extension, and by cross-linking hydrophilic pre-polymers or polymers. The present invention has recognized the ability to use a biocompatible hydrogel matrix in a cell cryopreservation method that overcomes the drawbacks described above.

A variety of hydrogels may be used according to the invention. In certain embodiments, as more fully described below in relation to a specific, non-limiting embodiment of the invention, a hydrogel for use in the present invention may be comprised of naturally occurring materials. In other embodiments, hydrogels including or based on synthetic polymers may also be used in the invention. Although the invention is particularly described in relation to a hydrogel formed of a polypeptide and a polyglycan, a skilled person using the disclosure of the invention would understand that the hydrogels formed using synthetic components could similarly be formed, lyophilized, made into particles, and used for cryopreservation, as described herein. The invention fully encompasses such a variety of hydrogels because, while not wishing to be bound by theory, it is believed that trapped water within the structure of a hydrogel provides a means of preventing ice crystallization and cell disruption in cells that are attached to the lyophilized particles.

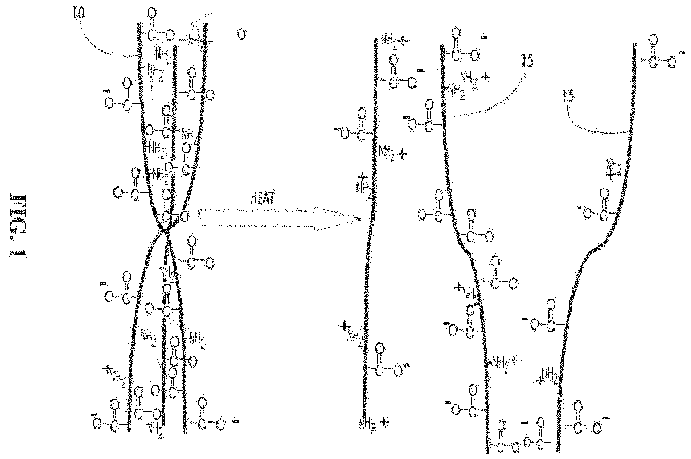

The formulation of a thermoreversible hydrogel matrix providing a cell culture medium and composition for preserving cell viability is taught by U.S. Patent No. 6,231,881 and U.S. Patent No. 6,730,315, both of which are herein incorporated by reference in their entirety. Additionally, a hydrogel matrix useful in promoting vascularization is provided in U.S. Patent No. 6,261,587, herein incorporated by reference in its entirety. Further, a hydrogel matrix useful in obscuring immune recognition is provided in U.S. Patent No. 6,352,707, herein incorporated by reference in its entirety. The present invention, in certain embodiments, likewise incorporates a thermoreversible hydrogel matrix that is a gel at storage temperatures and molten at physiologic temperatures, and comprises a combination of a collagen-derived component, such as gelatin, a long chain polyglycan, such as dextran, and effective amounts of other components, such as polar amino acids. The discussion below in relation to FIG. 1 through FIG. 3 describes the thermoreversible gel and provides a basis for the cross-linked hydrogel matrix of the invention. As incorporated into the present invention, the thermoreversible gel is not cross-linked and can encompass hydrogel components described herein absent the additional cross-linking associated with the more stable cross-linked gel.

In preferred embodiments, the hydrogel matrix comprises a polypeptide component and a polyglycan component. Polypeptides are generally understood to be a series of amino acids joined by peptide bonds between the $\alpha$-carboxyl group of one amino acid and the $\alpha$-amino group of the next amino acid in the series. Thus, a specific polypeptide is defined by its specific amino acid sequence.

A polypeptide, as used herein, is intended to encompass any tissue-derived or synthetically produced polypeptide, such as collagens or collagen-derived gelatins. In specific embodiments, a polypeptide according to the invention can comprise from about 50 amino acid residues to about 30,000 amino acid residues, preferably about 100 amino acid residues to about 20,000 amino acid residues, more preferably about 200 amino acid residues to about 10,000 amino acid residues, still more preferably about 300 amino acid residues to about 5,000 amino acid residues, and most preferably about 500 amino acid residues to about 2,000 amino acid residues.

Although collagen-derived gelatin is the preferred polypeptide component, other gelatin-like components characterized by a backbone comprised of sequences of amino acids having polar groups that are capable of interacting with other molecules can be used. For example, keratin, decorin, aggrecan, elastin, glycoproteins (including proteoglycans), and the like could be used to provide the polypeptide component. In one embodiment, the polypeptide component is porcine gelatin from partially hydrolyzed collagen derived from skin tissue. Polypeptides derived from other types of tissue could also be used. Examples include, but are not limited to, tissue extracts from arteries, vocal chords, pleura, trachea, bronchi, pulmonary alveolar septa, ligaments, auricular cartilage or abdominal fascia; the reticular network of the liver; the basement membrane of the kidney; or the neurilemma, arachnoid, dura mater or pia mater of the nervous system. Purified polypeptides including, but not limited to, laminin, nidogen, fibulin, and fibrillin or protein mixtures such as those described by U.S. Patent No. 6,264,992 and U.S. Patent No. 4,829,000, extracts from cell culture broth as described by U.S. Patent No. 6,284,284, submucosal tissues such as those described in U.S. Patent No. 6,264,992, or gene products such as described by U.S. Patent No. 6,303,765 may also be used. Another example of a suitable polypeptide is a fusion protein formed by genetically engineering a known reactive species onto a protein.

The polypeptide component preferably has a molecular mass range of about 3,000 to about 3,000,000 Da, more preferably about 30,000 to about 300,000 Da, most preferably about 50,000 to about 250,000 Da. Molecular mass can be expressed as a weight average molecular mass ($M_w$) or a number average molecular mass ($M_n$). Both expressions are based upon the characterization of macromolecular solute containing solution as having an average number of molecules ($n_i$) and a molar mass for each molecule ($M_i$). Accordingly, number average molecular mass is defined by formula 1 below.

$$M_n = \frac{\sum n_i M_i}{\sum n_i} \qquad (1)$$

Weight average molecular mass (also known as molecular mass average) is directly measurable using light scattering methods and is defined by formula 2 below.

$$M_w = \frac{\sum n_i M_i^2}{\sum n_i M_i} \qquad (2)$$

Molecular mass can also be expressed as a Z-average molar mass ($M_z$), wherein the calculation places greater emphasis on molecules with large molar masses. Z-average molar mass is defined by formula 3 below.

$$M_z = \frac{\sum n_i M_i^3}{\sum n_i M_i^2} \qquad (3)$$

Unless otherwise noted, molecular mass is expressed herein as weight average molecular mass.

In addition to molecular mass, polymer solutions can also be physically described in terms of polydispersity, which represents the broadness of the molecular mass distribution within the solution, such distribution being the range of different molecular masses of the individual polymer molecules in the solution. Polydispersity is the ratio of the number average molecular mass to the weight average molecular mass, which is defined by formula 4 below.

$$\text{Polydispersity} = \frac{M_w}{M_n} \qquad (4)$$

If polydispersity is equal to 1 (i.e., $M_n$ equals $M_w$), the polymer is said to be monodisperse. A truly monodisperse polymer is one where all polymer molecules within the solution are of a single, identical molecular mass. As $M_n$ changes with $M_w$, the polydispersity changes, always being greater than 1. The polydispersity of a given polymer solution can affect the physical characteristics of the polymer, and, therefore, the interaction of the polymer with another polymer. Research has shown that in aqueous mixtures of biopolymers (including gelatin and dextran), an increase in molecular weight results in a less compatible system with a higher phase separation temperature, whereas a decrease in concentration results in a more compatible system with a lower phase separation temperature (see E. H. A. de Hoog and R. H. Tromp, Colloids and Surfaces A: Physicochemical and Engineering Aspects, 213 (2-3), Pages 221-234). Preferably, the polypeptide used according to the present invention has a polydispersity close to 1. In one preferred embodiment, the polypeptide has a polydispersity of 1 to about 4, more preferably, about 1 to about 3, most preferably about 1.1 to about 2.4.

The polypeptide used in the bioactive hydrogel matrix of the invention is preferably a gelatin. In certain embodiments, the gelatin is derived from mammalian tissue. In a particularly preferred embodiment, the polypeptide comprises collagen-derived gelatin.

Collagen is a major protein component of the extracellular matrix of animals. Early in fetal development, a more open form of collagen (compared to tightly bound mature collagen) is associated with large carbohydrate molecules, and serves as the predominant tissue scaffolding. It is believed that attachment of differentiated or incompletely differentiated cells of mesenchymal origin to this polar, proteoglycan-like, collagen scaffolding results in a specific host tissue response. This response is to guide the differentiation of mesenchymal tissue.

Collagen is assembled into a complex fibrillar organization. The fibrils are assembled into bundles that form the fibers. The fibrils are made of five microfibrils placed in a staggered arrangement. Each microfibril is a collection of collagen rods. Each collagen rod is a right-handed triple-helix, each strand being itself a left-handed helix. Collagen fibrils are strengthened by covalent intra- and intermolecular cross-links which make the tissues of mature animals insoluble in cold water. When suitable treatments are used, collagen rods are extracted and solubilized where they keep their conformation as triple-helices. This is denatured collagen and differs from the native form of collagen, but has not undergone sufficient thermal or chemical treatment to break the intramolecular stabilizing covalent bonds found in collagen. When collagen solutions are extensively heated, or when the native collagen containing tissues are subjected to chemical and thermal treatments, the hydrogen and covalent bonds that stabilize the collagen helices are broken, and the molecules adopt a disordered conformation. By breaking these hydrogen bonds, the polar amine and carboxylic acid groups are now available for binding to polar groups from other sources or themselves. This material is gelatin and is water-soluble at 40-45°C.

As noted above, gelatin is a form of denatured collagen, and is obtained by the partial hydrolysis of collagen derived from the skin, white connective tissue, or bones of animals. Gelatin may be derived from an acid-treated precursor or an alkali-treated precursor. Gelatin derived from an acid-treated precursor is known as Type A, and gelatin derived from an alkali-treated precursor is known as Type B. The macromolecular structural changes associated with collagen degradation are basically the same for chemical and partial thermal hydrolysis. In the case of thermal and acid-catalyzed degradation, hydrolytic cleavage predominates within individual collagen chains. In alkaline hydrolysis, cleavage of inter- and intramolecular cross-links predominates.

Preferably, the gelatin used in the present invention is skin-derived gelatin or bone-derived gelatin. In one preferred embodiment, the gelatin has a molecular mass of about 80,000 Da to about 200,000 Da. Further, it is preferred that the gelatin have a polydispersity of 1 to about 3. In one preferred embodiment, the gelatin has a polydispersity of about 1.1 to about 2.4.

The polypeptide, such as gelatin, is preferentially present at a concentration of about 0.01 to about 40 mM, preferably about 0.05 to about 30 mM, most preferably about 0.25 to about 5 mM. Advantageously, the gelatin concentration is approximately 0.75 mM. The above concentrations provide a non-flowable phase at storage temperature (below about 33 °C) and a flowable phase at treatment temperature (about 35 to about 40 °C).

The bioactive hydrogel matrix of the present invention also comprises a long chain carbohydrate. The phrase long chain carbohydrate is generally intended to encompass any polyglycan (defined to encompass polysaccharides and sulfated polysaccharides) consisting of more than about 10 monosaccharide residues joined to each other by glycosidic linkages. The phrase is also intended to encompass other long chain carbohydrates, including heterosaccharides, and specific classes of carbohydrates, such as starches, sugars, celluloses, and gums. The long chain carbohydrate may consist of the same monosaccharide residues, or various monosaccharide residues or derivatives of monosaccharide residues. Dextran, a preferred polysaccharide, solely comprises glucose residues.

Any polysaccharide, including glycosaminoglycans (GAGs) or glucosaminoglycans, with suitable viscosity, molecular mass and other desirable properties may be utilized in the present invention. By glycosaminoglycan is intended any glycan (*i.e.,* polysaccharide) comprising an unbranched polysaccharide chain with a repeating disaccharide unit, one of which is always an amino sugar. These compounds as a class carry a high negative charge, are strongly hydrophilic, and are commonly called mucopolysaccharides. This group of polysaccharides includes heparin, heparan sulfate, chondroitin sulfate, dermatan sulfate, keratan sulfate, and hyaluronic acid. These GAGs are predominantly found on cell surfaces and in the extracellular matrix. By glucosaminoglycan is intended any glycan (*i.e.* polysaccharide) containing predominantly monosaccharide derivatives in which an alcoholic hydroxyl group has been replaced by an amino group or other functional group such as sulfate or phosphate. An example of a glucosaminoglycan is poly-N-acetyl glucosaminoglycan, commonly referred to as chitosan. Exemplary polysaccharides that may be useful in the present invention include dextran, heparan, heparin, hyaluronic acid, alginate, agarose, carrageenan, amylopectin, amylose, glycogen, starch, cellulose, chitin, and chitosan. Derivatized polysaccharides may also be used, including but not limited to sulfates, acetates, phosphates, and ammonium salts of polysaccharides. For example, various sulfated polysaccharides such as heparan sulfate, chondroitin sulfate, dextran sulfate, dermatan sulfate, or keratan sulfate could be used.

The long chain carbohydrate preferably has a molecular mass of about 2,000 to about 8,000,000 Da, more preferably about 20,000 to about 1,000,000 Da, most preferably about 200,000 to about 800,000 Da. In one embodiment, the long chain carbohydrate has a molecular mass of approximately 500,000 Da.

Preferably, the long chain carbohydrate used according to the present invention has a polydispersity close to 1. In one preferred embodiment, the polypeptide has a polydispersity of 1 to about 3, more preferably, about 1.1 to about 2.4.

As previously noted, one preferred long chain carbohydrate for use in the present invention is dextran. Dextran typically comprises linear chains of $\alpha(1\rightarrow6)$-linked D-glucose residues, often with $\alpha(1\rightarrow2)$- or $\alpha(1\rightarrow3)$-branches. Native dextran, produced by a number of species of bacteria of the family Lactobacilliaceae, is a polydisperse mixture of components. Dextrans have been widely used as plasma substitutes and blood extenders, are considered fully biocompatible, and are metabolizable. Dextrans are available in a wide range of average molecular masses, varying from about 4,000 to about 40,000,000 Da. Preferably, the dextran used in the invention has a molecular mass of about 200,000 to about 800,000 Da, most preferably about 300,000 to about 600,000 Da. In one preferred embodiment, the dextran has a molecular mass of approximately 500,000 Da. Dextrans have varying rates of resorption *in vivo* from about two to about 20 days depending on their molecular mass.

The long chain carbohydrate, such as dextran, is preferentially present at a concentration of about 0.01 to about 10 mM, preferably about 0.01 to about 1 mM, most preferably about 0.01 to about 0.5 mM. In one embodiment, dextran is present at a concentration of about 0.1 mM.

While native dextran is generally used in the present invention, the use of dextran derivatives, such as dextran sulfate and dextran phosphate is also within the scope of the invention. In one embodiment, the derivatives are free radical polymerizable, preferably photopolymerizable derivatives, such as acrylates. According to this embodiment, the composition can be injected as a viscous liquid and polymerized in situ to form a solid material. The dextran can also be selected to degrade at a rate which approximates ingrowth of new bone or tissue. Those compositions that include free radical polymerizable groups may also include polymerization initiators, such as photoinitiators, such as benzoin ethers, and thermally activatable initiators, such as azobisisobutyronitrile (AIBN) and di-t-butyl ether. Free radical polymerization initiators, and conditions for carrying out free radical polymerizations, are well known to those of skill in the art, and any of such methods are encompassed by the present invention.

In a preferred embodiment, gelatin and dextran are components of the bioactive hydrogel matrix of the present invention. For ease of describing the invention, the terms "gelatin" and "dextran" are used throughout with the understanding that various alternatives as described above, such as other polypeptides and other long chain carbohydrates readily envisioned by those skilled in the art, are contemplated by the present invention.

Although not bound by any particular theory, the present invention is intended to provide a matrix scaffolding designed to maximize the polar amino acid hydrogen bonding sites found in alpha chains derived from collagen. These alpha chains, or gelatin, are preferably derived from pig gelatin, and stabilized by 500,000 Da molecular mass dextran, or other long chain carbohydrates, added while the alpha chains are heated. The positively charged polar groups of the collagen-derived alpha chains are then able to associate with the negatively charged -OH groups of the repeating glucose units found in the dextran. The gelatin and the dextran form a proteoglycan-type structure. Figures 1-3 illustrate the interaction between the various components of the preferred embodiment of the matrix of the invention and interaction between the matrix and the tissue of a patient.

FIG. 1 illustrates the creation of polar alpha chains **15** from tropocollagen **10** derived from mature collagen. Heating tropocollagen **10** disrupts the hydrogen bonds that tightly contain the triple stranded monomers in mature collagen. By breaking these hydrogen bonds, the polar amine and carboxylic acid groups are now available for binding to polar groups from other sources or themselves.

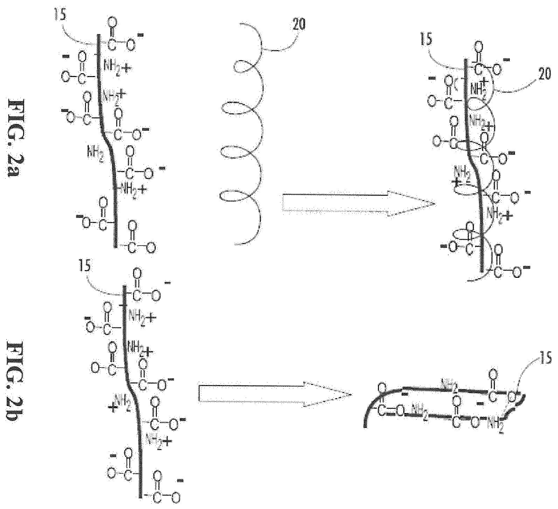

FIG. 2A illustrates stabilization of the matrix monomeric scaffolding by the introduction of a long chain carbohydrate **20,** such as dextran. As shown in FIG. 2B, without the long chain carbohydrate **20,** the alpha chain **15** will form hydrogen bonds between the amino and carboxylic acid groups within the linear portion of the monomer and fold upon itself, thus limiting available sites for cellular attachment. As depicted in FIG. 2A, the long chain carbohydrate **20** serves to hold the alpha chain **15** open by interfering with this folding process.

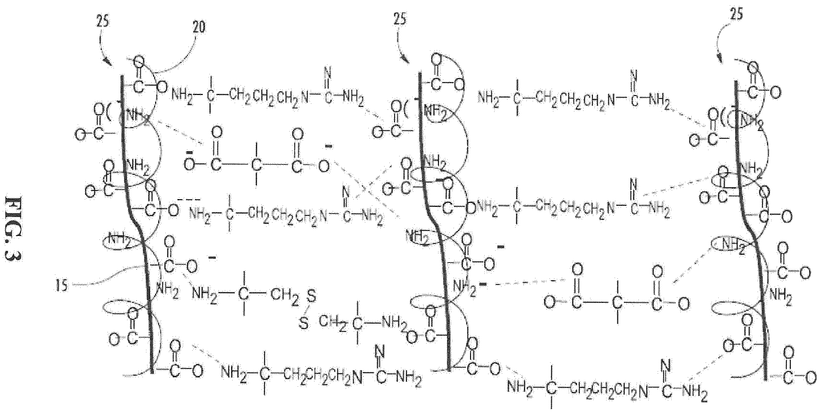

FIG. 3 illustrates the effect of polar amino acids and/or L-cysteine added to stabilize the monomer/carbohydrate units **25** by linking the exposed monomer polar sites to, for example, arginine's amine groups or glutamic acid's carboxylic acid groups. Furthermore, disulfide linkages can be formed between L-cysteine molecules (thereby forming cystine), which in turn forms hydrogen bonds to the monomeric alpha chains **15.** The stability imparted by the polar amino acids, polar amino acid analogues and derivatives, and intact collagen is particularly advantageous for maintaining the open structure of the gelatin and keeping the active sites available for therapeutic benefit.

The hydrogen bonds formed between these additional amino acids and monomer/carbohydrate units **25** are broken when the matrix is liquefied upon heating, and the polar groups are freed to attach the monomer/dextran units to exposed patient tissue surfaces. In preferred embodiments, EDTA or a salt thereof is also present to chelate divalent cations and thereby prevent divalent cations from being preferentially attracted to the exposed polar groups of the monomer/carbohydrate units **25** to the exclusion of the polar amino acids.

The alpha chain/dextran units **25** can serve as scaffolding on which host cells, such as stem cells, can integrate. These cells can then activate areas of their own genome that leads to differentiation into cell types required for tissue healing and regeneration. For example, SAOS 2 cells grown on the scaffolding have been shown to demonstrate a significant up-regulation of BMP2 gene expression important for bone repair.

By providing a proteoglycan-like scaffolding similar to that found in the early stages of fetal development, and using structural stabilizers that serve a secondary purpose in enhancing host response to the scaffolding upon exposure to host tissues, the matrix serves as a biocompatible device capable of increasing vascularization and promoting wound repair and local tissue regeneration.

In addition to the polypeptide and long chain carbohydrate, the bioactive hydrogel matrix can further comprise one or more components useful for enhancing the bioadhesiveness of the hydrogel matrix. As noted previously, the stabilized cross-linked hydrogel matrix of the present invention may be further stabilized and enhanced through the addition of one or more enhancing agents. By "enhancing agent" or "stabilizing agent" is intended any compound added to the hydrogel matrix in addition to the high molecular weight components that enhances the hydrogel matrix by providing further stability or functional advantages. Examples of such components include polar amino acids, polar amino acid analogues, polar amino acid derivatives, divalent cation chelators, and combinations thereof. In one preferred embodiment, all of the bioactive hydrogel matrix ingredients are provided in admixture. The enhancing agent can include any compound, especially polar compounds, that, when incorporated into the cross-linked hydrogel matrix, enhance the hydrogel matrix by providing further stability or functional advantages.

Preferred enhancing agents for use with the stabilized cross-linked hydrogel matrix include polar amino acids, analogues of amino acids (particularly polar amino acid analogues), amino acid derivatives (particularly polar amino acid derivatives), intact collagen, and divalent cation chelators, such as ethylenediaminetetraacetic acid (EDTA) or salts thereof. The group of "polar amino acids" is intended to include tyrosine, cysteine, serine, threonine, asparagine, glutamine, aspartic acid, glutamic acid, arginine, lysine, and histidine. The preferred polar amino acids are L-cysteine, L-glutamic acid, L-lysine, and L-arginine. Suitable concentrations of each particular preferred enhancing agent are the same as noted above in connection with the thermoreversible hydrogel matrix. Polar amino acids, EDTA, and mixtures thereof, are preferred enhancing agents. The enhancing agents can be added to the matrix composition before or during the crosslinking of the high molecular weight components.

The enhancing agents are particularly important in the stabilized cross-linked bioactive hydrogel matrix because of the inherent properties they promote within the matrix. The hydrogel matrix exhibits an intrinsic bioactivity that will become more evident through the additional embodiments described hereinafter. It is believed the intrinsic bioactivity is a function of the unique stereochemistry of the cross-linked macromolecules in the presence of the enhancing and strengthening polar amino acids, as well as other enhancing agents.

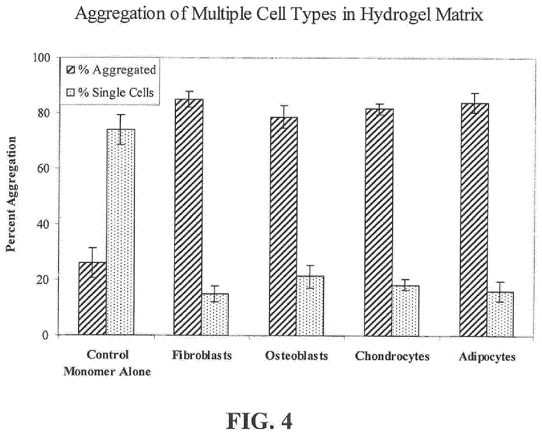

For example, aggregation of human fibroblasts exposed to bioactive hydrogels has been observed, while aggregation is not observed when fibroblasts are exposed to the individual components of the bioactive hydrogel. Results from numerous (over fifty) controlled experiments have shown that normal neonatal human skin fibroblasts form multi-cell aggregates when exposed to the complete thermoreversible hydrogel formulation at 37 °C, while no such cell aggregating activity is demonstrated using omission formulations in which the bioactive copolymer is not formed. The aggregated cells form tightly apposed cell clusters with interdigitating cytoplasmic processes, while cells treated with formulations lacking the copolymer remain round and without surface projections. As shown in FIG. 4, in a sample of human fibroblasts exposed to a bioactive hydrogel comprising dextran and gelatin, at least 80% of the cells present were in an aggregated state while less than 20% of the cells present remained as single cells. The opposite effect was observed in samples where the human fibroblasts were exposed to collagen monomer alone, carbohydrate alone, or were left untreated. In samples exposed to collagen monomer alone, approximately 75% of the cells remained in a single cell configuration while only about 25% of the cells were in an aggregated state. Nearly the same effect was observed in samples exposed to carbohydrate alone. In samples that were left untreated, approximately 60% of the cells remained in a single cell state while only about 40% of the cells were in an aggregated state.

As used herein, polar amino acids are commonly defined and intended to include tyrosine, cysteine, serine, threonine, asparagine, glutamine, aspartic acid, glutamic acid, arginine, lysine, and histidine. Preferentially, the amino acids are selected from the group consisting of cysteine, arginine, lysine, histidine, glutamic acid, and aspartic acid. When polar amino acids are present in the bioactive hydrogel matrix, the polar amino acids are preferentially present in a concentration of about 3 to about 150 mM, preferably about 10 to about 65 mM, and more preferably about 15 to about 40 mM.

Advantageously, the added polar amino acids comprise L-glutamic acid, L-lysine, and L-arginine. The final concentration of L-glutamic acid is generally about 2 to about 60 mM, preferably about 5 to about 40 mM, most preferably about 10 to about 30 mM. In one embodiment, the concentration of L-glutamic acid is about 20 mM. The final concentration of L-lysine is generally about 0.5 to about 30 mM, preferably about 1 to about 15 mM, most preferably about 1 to about 10 mM. In one embodiment, the concentration of L-lysine is about 5.0 mM. The final concentration of L-arginine is generally about 1 to about 40 mM, preferably about 1 to about 30 mM, most preferably about 5 to about 20 mM. In one embodiment, the final concentration of arginine is about 15 mM.

By amino acid is intended all naturally occurring alpha amino acids in both their D and L stereoisomeric forms, and their analogues and derivatives. An analog is defined as a substitution of an atom or functional group in the amino acid with a different atom or functional group that usually has similar properties. A derivative is defined as an amino acid that has another molecule or atom attached to it. Derivatives would include, for example, acetylation of an amino group, amination of a carboxyl group, or oxidation of the sulfur residues of two cysteine molecules to form cystine. As previously noted, the bioactive hydrogel matrix of the invention can include one or more polar amino acid analogues or derivatives.

Amino acids used in the bioactive hydrogel matrix of the present invention can also be present as dipeptides, which are particular beneficial for delivery of amino acids having decreased water solubility, such as L-glutamine. Accordingly, amino acids added to the hydrogel matrix can include dipeptides, such as L-alanyl-L-glutamine. When present in the hydrogel matrix, the concentration range for L-alanyl-L-glutamine is preferably about 0.001 to about 1 mM, more preferably about 0.005 to about 0.5 mM, most preferably about 0.008 to about 0.1 mM. In one particular embodiment, the final concentration of L-alanyl-L-glutamine is about 0.01 mM.

The added amino acids can also include cysteine (such as L-cysteine), which is advantageous in many regards. Cysteine is useful for providing disulfide bridges, further adding support and structure to the bioactive hydrogel matrix and increasing its resistance to force. The final concentration of cysteine is generally about 5 to about 5000 $\mu$M, preferably about 10 to about 1000 $\mu$M, most preferably about 100 to about 1000 $\mu$M. In one embodiment, the final concentration of cysteine is about 700 $\mu$M. L-cysteine also acts as a nitric oxide scavenger or inhibitor. Nitric oxide inhibitors include any composition or agent that inhibits the production of nitric oxide or scavenges or removes existing nitric oxide. Nitric oxide, a pleiotropic mediator of inflammation, is a soluble gas produced by endothelial cells, macrophages, and specific neurons in the brain, and is active in inducing an inflammatory response. Nitric oxide and its metabolites are known to cause cellular death from nuclear destruction and related injuries.

Accordingly, the bioactive hydrogel matrix can optionally include one or more additional nitric oxide inhibitors, such as N-monomethyl-L-arginine, N-nitro-L-arginine, cysteine, heparin, and mixtures thereof. When present in the hydrogel matrix, the final concentration of nitric oxide inhibitors is generally about 5 to about 500 $\mu$M, preferably about 10 to about 100 $\mu$M, most preferably about 15 to about 25 $\mu$M. In one embodiment, the final concentration is about 20 $\mu$M.

Advantageously, intact collagen can be optionally added to the bioactive hydrogel matrix to provide an additional binding network and provide additional support to the matrix. The final concentration of the intact collagen present in the hydrogel matrix is from about 0 to about 5 mM, preferably about 0 to about 2 mM, most preferably about 0.05 to about 0.5 mM.

Additionally, the bioactive hydrogel matrix may optionally include one or more divalent cation chelators, which increase the rigidity of the matrix by forming coordinated complexes with any divalent metal ions present. The formation of such complexes leads to the increased rigidity of the matrix by removing the inhibition of hydrogen bonding between -NH$_2$ and -COOH caused by the presence of the divalent metal ions. A preferred example of a divalent cation chelator that is useful in the present invention is ethylenediaminetetraacetic acid (EDTA) or a salt thereof. The concentration range for the divalent cation chelator, such as EDTA, is generally about 0.01 to about 10 mM, preferably 1 to about 8 mM, most preferably about 2 to about 6 mM. In a one embodiment, EDTA is present at a concentration of about 4 mM.

EDTA is also an example of another group of compounds useful as additives for the bioactive hydrogel matrix, superoxide inhibitors. Superoxide is a highly toxic reactive oxygen species, whose formation is catalyzed by divalent transition metals, such as iron, manganese, cobalt, and sometimes calcium. Highly reactive oxygen species such as superoxide ($O_2^-$) can be further converted to the highly toxic hydroxyl radical ($OH^-$) in the presence of iron. By chelating these metal catalysts, EDTA serves as an antioxidant. Accordingly, the bioactive hydrogel matrix can include one or more superoxide inhibitor.

The bioactive hydrogel matrix is preferably based upon a physiologically compatible buffer, one embodiment being Medium 199, a common nutrient solution used for *in vitro* culture of various mammalian cell types (available commercially from Sigma Chemical Company, St. Louis, MO). The buffer can be further supplemented with additives and additional amounts of some medium components, such as supplemental amounts of polar amino acids as described above.

The bioactive hydrogel matrix can also be formulated in other buffered solutions, including buffered solutions regarded as simplified in relation to Medium 199. For example, a phosphate buffer formulated to yield physiological osmotic pressures after hydrogel matrix compounding can be prepared using 1.80 mM KH$_2$PO$_4$ and 63 mM Na$_2$HPO$_4$.

*In vitro* testing has shown that the bioactive hydrogel matrix of the invention exhibits a remarkable ability to bind to and hence promote cell aggregation across multiple cell types. Treatment of cultured osteoblasts (human osteosarcoma cell line SAOS-2) with the bioactive hydrogel matrix resulted in approximately 80% cellular aggregation. In one comparative study, cells were treated with the bioactive hydrogel matrix of the invention, and cells (control) were treated with gelatin alone. Cell types tested were fibroblasts, osteoblasts, chondrocytes, and adipocytes. The cells were stained with toluidine and visually inspected. The cells treated with the bioactive hydrogel matrix were evident as large clumps (*i.e.,* aggregates), while the control cells (those treated with gelatin alone) were evident as single cells and not aggregated. This illustrates how the intact bioactive hydrogel matrix binds to and aggregates cells important in wound healing, bone repair, and non-bone connective tissue repair. This binding and subsequent interaction does not occur when only gelatin is present. Furthermore, previous similar studies with fibroblasts indicated the binding and aggregation also did not occur after treatment with dextran alone.

The present invention comprises a cross-linked hydrogel matrix that is both bioactive and stabilized. By "bioactive" is intended the ability to facilitate or discourage a cellular or tissue response of a host to foreign materials introduced to the body. Examples include, but are not limited to, induction of vasculogenesis, inhibition of the formation of a foreign body response, promotion of cellular attachment to the scaffold material, and promotion of tissue regeneration. The term "stabilized" or "stable", as used herein, is intended to refer to compositions that are water-swellable, poorly soluble, solid or semi-solid materials at physiological temperature (i.e., about 37°C) and in physiological fluids (e.g.,

aqueous body fluids having a physiological pH of about 7.4), which remain present in the host for sufficient time to achieve the intended response. Such a stabilized, bioactive, cross-linked hydrogel matrix is similarly disclosed in U.S. Patent Application Publication No. 2003/0232746, which is incorporated herein by reference in its entirety.

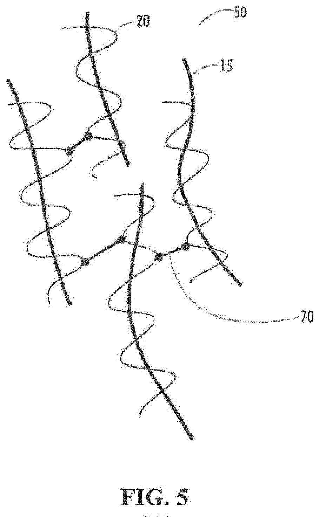

In one embodiment of the present invention, as illustrated in FIG. 5, dextran **20** is covalently crosslinked to gelatin **15** by linkages **70,** thereby forming a crosslinked network **50**. The linkages **70** either result from reaction of functional groups on the gelatin **15** with functional groups on the dextran **20,** or result from reaction of a bifunctional crosslinker molecule with both the dextran **20** and gelatin **15**. As explained in greater detail below, one method of crosslinking gelatin and dextran is to modify the dextran molecules **20,** such as by oxidation, in order to form functional groups suitable for covalent attachment to the gelatin **15**. This stabilized cross-linked bioactive network **50** yields therapeutically useful gels and pastes that are insoluble in physiologic fluids at physiological temperatures. No additional substrate or surface is required. The so-formed gels and pastes are appropriate for the development of therapeutic methods based on the induction of a localized vasculogenesis, wound healing, tissue repair, and regeneration. The bioactive hydrogel matrix particularly can be lyophilized and particularized to provide a granular or powder form of the cross-linked matrix, as described more fully below, while still retaining its other intrinsic activities related to wound healing and vasculogenesis, as described herein.

In one embodiment of a method of making the cross-linked hydrogel matrix, one of the high molecular weight components must be modified to form reactive groups suitable for cross-linking. For instance, the dextran or other polyglycan component can be modified, such as by oxidation, in order to cross-link with the gelatin component. One known reaction for oxidizing polysaccharides is periodate oxidation. The basic reaction process utilizing periodate chemistry is well known and appreciated by those skilled in the art. Periodate oxidation is described generally in Affinity Chromatography: A Practical Approach, Dean, et al., IRL Press, 1985 ISBN0-904147-71-1, which is incorporated by reference in its entirety. The oxidation of dextran by the use of periodate-based chemistry is described in U.S. Patent No. 6,011,008, which is herein incorporated by reference in its entirety.

In periodate oxidation, polysaccharides may be activated by the oxidation of the vicinal diol groups. With polyglycans, this is generally accomplished through treatment with an aqueous solution of a salt of periodic acid, such as sodium periodate ($NaIO_4$), which oxidizes the sugar diols to generate reactive aldehyde groups (e.g. dialdehyde residues). This method is a rapid, convenient alternative to other known oxidation methods, such as those using cyanogen bromide. Polyglycans activated by periodate oxidation may be stored at 4°C for several days without appreciable loss of activity.

Polyglycan materials, such as dextran, activated in this manner readily react with materials containing amino groups, such as gelatin, producing a cross-linked material through the formation of Schiff's base links. A Schiff base is a name commonly used to refer to the imine formed by the reaction of a primary amine with an aldehyde or ketone. The aldehyde groups formed on the cellulosic surface react with most primary amines between pH values from about 4 to about 6. The Schiff's base links form between the dialdehyde residues of the polyglycan and the free amino groups on the protein. The cross-linked product may subsequently be stabilized (i.e. formation of stable amine linkages) by reduction with a borohydride, such as sodium borohydride ($NaBH_4$) or sodium cyanoborohydride ($NaBH_3CN$). The residual aldehyde groups may be consumed with ethanolamine or other amine containing species to further modify the cross-linked matrix. Other methods known to those skilled in the art may be utilized to provide reactive groups on either one or both of the high molecular weight components of the matrix.

In the present invention, periodate chemistry is used with dextran to form a multifunctional polymer that can then react with gelatin and enhancing agents present during the manufacturing process. The periodate reaction leads to the formation of polyaldehyde polyglycans that are reactive with primary amines. For example, high molecular weight polypeptides and high molecular weight polyglycans may form covalent hydrogel complexes that are colloidal or covalently cross-linked gels. Covalent bonding occurs between reactive groups of the dextran and reactive groups of the gelatin component. The reactive sites on the gelatin include amine groups provided by arginine, asparagine, glutamine, and lysine. These amine groups react with the aldehyde or ketone groups on the dextran to form a covalent bond. These hydrogels can be readily prepared at temperatures from about 34°C to about 90°C. Additionally, the hydrogels can be prepared at a pH range of from about 5 to about 9, preferably from about 6 to about 8, and most preferably from about 7 to about 7.6.

By controlling the extent of dextran activation and the reaction time, one can produce stabilized biomimetic scaffolding materials of varying viscosity and stiffness. By "biomimetic" is intended compositions or methods imitating or simulating a biological process or material. Some biomimetic processes have been in use for several years, such as the artificial synthesis of vitamins and antibiotics. More recently, additional biomimetic applications have been proposed, including nanorobot antibodies that seek and destroy disease-causing bacteria, artificial organs, artificial arms, legs, hands, and feet, and various electronic devices. The biomimetic scaffolding materials of the present invention yield therapeutically useful gels and pastes that are stable at about 37°C, or body temperature. These gels are capable of expansion and/or contraction, but will not dissolve in aqueous solution.

As an alternate method for forming the crosslinked dextran/gelatin network, a multifunctional cross-linking agent may be utilized as a reactive moiety that covalently links the gelatin and dextran chains. Such bifunctional cross-linking agents may include glutaraldehyde, epoxides (e.g., bis-oxiranes), oxidized dextran, p-azidobenzoyl hydrazide, N-[α-maleimidoacetoxy]succinimide ester, p-azidophenyl glyoxal monohydrate, bis-[β-(4-azidosalicylamido)ethyl]disulfide, bis[sulfosuccinimidyl]suberate, dithiobis[succinimidyl proprionate, disuccinimidyl suberate, 1-ethyl-3-[3-dimethylaminopropyl]carbodiimide hydrochloride, and other bifunctional cross-linking reagents known to those skilled in the art.

In another embodiment utilizing a cross-linking agent, polyacrylated materials, such as ethoxylated (20) trimethylpropane triacrylate, may be used as a non-specific photo-activated cross-linking agent. Components of an exemplary reaction mixture would include a thermoreversible hydrogel held at 39 °C, polyacrylate monomers, such as ethoxylated (20) trimethylpropane triacrylate, a photo-initiator, such as eosin Y, catalytic agents, such as 1-vinyl-2-pyrrolidinone, and triethanolamine. Continuous exposure of this reactive mixture to long-wavelength light (> 498 nm) would produce a cross-linked hydrogel network.

The bioactive hydrogel matrix of the invention is itself useful in various therapeutic methods, such as site-specific tissue regeneration, including vasculogenesis. It is known in the art to use intact collagen, gelatin, or dextran as a carrier to hold and deliver growth factors and the like in methods designed to promote tissue growth. (See, for example, Kawai, K. et al., Biomaterials 21:489-499 (2000); and Wissink, M.J.B. et al., Biomaterials 22:2291-2299 (2001)). By contrast, the intrinsic activity of the stabilized cross-linked hydrogel of the present invention is sufficient to elicit a specific sequence of biological responses, such as promoting tissue regeneration and vasculogenesis, without the addition of exogenous drugs or growth factors. In fact, the cross-linked matrix of the invention can be substantially free, even completely free, of exogenous drugs or growth factors when used for vascularization or tissue regeneration. This intrinsically bioactive hydrogel, as a result of its unique structure, provides a cell attachment scaffold that modulates subsequent cellular activity, such as tissue regeneration and vasculogenesis. It is this intrinsic bioactivity and provision of a cell attachment scaffold that makes the cross-linked hydrogel matrix useful in cell cryopreservation according to the invention. The matrix provides a) a scaffold for the immobilized cells and b) a bioactive hydrogel for rapid vascularization at the site of implant.

It is believed that the stabilized cross-linked hydrogel of the present invention is useful as a wound healing device due to the intrinsic bioactivity of the material and the unique stereochemistry of the macromolecules in the presence of enhancing and strengthening polar amino acids. Several studies indicate the wound healing properties of collagen are attributable to its unique structure (see, Brass, L.F. and Bensusan, H., The Journal of Clinical Investigation 54:1480-1487 (1974); Jaffe, R. and Dykin, D., Journal of Clinical Investigation 53:875-883 (1974); Postlewaithe, A.E. and Kang, A.H., The Journal of Experimental Medicine 143:1299-1307 (1976); and Reddi, A.H., Biochemistry of Collagen, New York; Plenum Press, 449-477 (1976)). Similarly, the hydrogel of the present invention demonstrates unique activity as a scaffold for delivery of cells and as an intrinsic attractant for tissue building components and factors necessary to promote wound healing and cell integration. Moreover, the bioactive hydrogel matrix exhibits rapid mechanical integration in vivo.

The bioactive cross-linked hydrogel matrix utilized in each of the embodiments described herein may be comprised solely of the two high molecular weight components cross-linked to one another. Preferably, each of the embodiments described herein may incorporate additional components such as the enhancing agents utilized in the preferred embodiments described above. Table 1 below lists preferred components present within the stabilized cross-linked hydrogel matrix of the present invention along with suitable concentrations as well as preferred concentrations for each component. Note that the concentrations listed in Table 1 for gelatin and dextran would also be suitable for alternative polyglycan and polypeptide components.

Table 1

| Component | Concentration Range | Preferred Concentration |
| --- | --- | --- |
| L-glutamic acid | 2 to 60 mM | 15 mM |
| L-lysine | 0.5 to 30 mM | 5.0 mM |
| Arginine | 1 to 40 mM | 10 mM |
| Gelatin | 0.01 to 40 mM | 2 mM |
| L-cysteine | 5 to 500 μM | 20 μM |
| EDTA | 0.01 to 10 mM | 4 mM |
| Dextran (oxidized & native forms) | 0.01 to 10 mM | 0.1 mM |

Of course, as previously pointed out, synthetic materials may also be used to form a hydrogel for use in the present invention. For example, the excellent biocompatibility of polyethylene glycol (PEG) and chemical derivatizations of PEG end groups, PEG and PEG-based copolymer hydrogels may be particularly useful. PEG containing terminal acrylates and α-hydroxy acids can be photo-polymerized to form hydrogels. Cross-linkers containing peptide sequences can be introduced into such gels. Peptides containing terminal cysteines can crosslink branched or multiarmed (or star-shaped) PEG upon simple mixing and form hydrogels. PEG hydrogels can also be modified with sugar residues to enhance adhesion to certain cell types. Other synthetic materials that can be used, optionally in combination with PEG, include polylactic acid (PLA), polyvinyl alcohol (PVA), polyacrylates, polymethacrylates, and the like.

The cross-linked hydrogel matrix of the present invention is preferably provided in a particulate form. Any method recognized in the art for particularizing a hydrogel can be used to provide a particulate hydrogel matrix according to the present invention. In one embodiment, the hydrogel matrix is dehydrated, such as by lyophilization, and then milled to form particles. A lyophilized, particulate hydrogel matrix is similarly disclosed in U.S. Patent Application Publication 2005/0118230, incorporated herein by reference in its entirety.

One preferred method of dehydrating the bioactive hydrogel matrix is freeze drying. Other methods of preparing dehydrated biopolymers, such as spray-drying or speed-vac, can also be used and are known to those skilled in the art.

Freeze drying generally comprises the removal of water or other solvent from a frozen product through sublimation, which is the direct transition of a material (e.g., water) from a solid state to a gaseous state without passing through the liquid phase. Freeze drying allows for the preparation of a stable product being readily re-hydratable, easy to use, and aesthetic in appearance. The freeze drying process typically consists of three stages: 1) pre-freezing, 2) primary drying, and 3) secondary drying.

Since freeze drying involves a phase change from solid to gaseous, material for freeze drying must first be adequately pre-frozen. The pre-freezing method and the final frozen product temperature can both affect the ability to successfully freeze dry the material. Rapid cooling forms small ice crystals. While small crystals are useful in preserving structure, they result in a product that is more difficult to freeze dry. Slower cooling results in larger ice crystals and produces less restrictive channels in the matrix during the drying process. Pre-freezing to temperatures below the eutectic temperature, or glass transition temperature, is necessary for complete drying of hydrogels. Inadequate freezing may produce small pockets of unfrozen material remaining in the product which may expand and compromise the structural stability of the freeze dried product.

After pre-freezing the product, conditions must be established in which ice (*i.e.,* frozen solvent) can be removed from the frozen product via sublimation, resulting in a dry, structurally intact product. This requires careful control of the two parameters, temperature and pressure, involved in the freeze drying system. It is important that the temperature at which a product is freeze dried is balanced between the temperature that maintains the frozen integrity of the product and the temperature that maximizes the vapor pressure of the solvent.

After primary freeze drying is complete, and all ice has sublimed, bound moisture is still present in the product. The product appears dry, but the residual moisture content may be as high as 7-8%. Continued drying is necessary at a warmer temperature to reduce the residual moisture content to optimum values. This process is called isothermal desorption, as the bound water is desorbed from the product. This secondary drying is normally continued at a product temperature higher than ambient but compatible with the sensitivity of the product. All other conditions, such as pressure and collector temperature, remain the same. Because the process is desorptive, the vacuum should be as low as possible (no elevated pressure) and the collector temperature as cold as can be attained. Secondary drying is usually carried out for approximately 1/3 to 1/2 the time required for primary drying.

One example of equipment useful in preparing freeze dried hydrogels is the FreeZone 12 Liter Freeze Dry System with Stoppering Tray Dryer (Labconco, Kansas City, MO). With such system, tubes with porous caps containing hydrogels are frozen to -30 °C at a cooling rate of 0.05 °C/min using the cooling shelf unit of the freeze dryer and are held at -30 °C for 12 hours. A vacuum is applied to the frozen hydrogel at -30 °C for 24 hours before the temperature is incrementally increased to -10 °C at a rate of 0.25 °C/minute. The hydrogel is held under vacuum at -10 °C for at least 12 hours before the temperature is further increased to 20 °C at a rate of 0.05 °C/minute.

The dehydrated hydrogel matrix can be made into a particulate form using any method recognized as useful in the art. For example, the lyophilized hydrogel matrix could be subjected to milling, such as using a grinding mill, ball mill, rod mill, impact mill, jet mill, vortex mill, or any other like apparatus recognized in the art as useful for forming a particulate material. With the exception of the jet and vortex mill, in order to obtain particle comminution, most mills rely on an interaction between the particulate solid and another surface, such as the balls in a ball mill, or a baffle or impact surface in an impact mill.

Milling and other methods of forming particulates generally provide a product having a range of particle sizes. Accordingly, in certain embodiments, it is preferred to further process the particulate to size the particles, such as by sieving. Preferentially, the particulate hydrogel matrix used in the present invention has an average particle size within a specified range.

Average particle size can be described in relation to a standard U.S. mesh sieve size. For example, in certain embodiments, greater than 90% of the particles pass through a 10 mesh screen, and greater than 90% of the particles are retained on a 100 mesh screen. In another embodiment, greater than 90% of the particles pass through an 18 mesh screen, and greater than 90% of the particles are retained on an 80 mesh screen. In a further embodiment, greater than 90% of the particles pass through a 25 mesh screen, and greater than 90% of the particles are retained on a 70 mesh screen. In still another embodiment, greater than 90% of the particles pass through a 30 mesh screen, and greater than 90% of the particles are retained on a 50 mesh screen.

Average particle size can further be described in relation to the absolute dimensions of the particles. As the particles are typically irregular in shape, size herein describes the largest dimension of an individual particle. In certain embodiments, the hydrogel matrix particles have an average particle size of about 0.01 mm to about 2 mm. In further embodiments, the hydrogel matrix particles have an average particle size of about 0.05 mm to about 1.5 mm, about 0.1 mm to about 1 mm, about 0.15 mm to about 0.9 mm, or about 0.25 mm to about 0.85 mm.

The particulate hydrogel matrix is useful as a substrate for attachment of cells. Accordingly, in further embodiments, the particulate hydrogel matrix can be described in relation to the number of cells retained per particle. In preferred embodiments, the particulate hydrogel matrix has an average particle size sufficient to allow for an individual particle to retain (e.g., facilitate attachment of) an average of at least about 20 cells, preferably at least about 50 cells, and more preferably at least about 100 cells. In further embodiments, the particles retain (e.g., facilitate attachment of) an average of about 10 cells to about 200 cells, preferably about 10 cells to about 150 cells, and more preferably about 20 cells to about 100 cells. It is particularly beneficial for the hydrogel matrix particles to be capable of supporting a number of cells that is sufficient to carry out the enhancement of a healing response at a tissue site to be treated. Moreover, as discussed later, seeding of cells onto the particles can be followed by *in vitro* culture to allow time for cell recovery from harvest and to further populate the fragments, including the establishment of networks of cooperating cells. Thus, it is further beneficial for the particles to be capable of allowing for attachment of cells, as well as growth of new cells during an incubation period.

Cell attachment to the hydrogel matrix particles can depend upon the overall surface area of the individual particles. This is partially dictated by the particle size, as described above. Surface area, however, is also partially dependant upon the overall porosity of the particulate hydrogel matrix.

The particulate hydrogel matrix is particularly characterized by its porous nature, which can advantageously be controlled to provide desirable characteristics and activities. Porosity of crosslinked hydrogels can be controlled by a variety of methods, such as: (a) the introduction of pore-forming agents or the use of foaming agents; (b) reducing the extent of hydrogel cross-linking; (c) reducing the concentration of hydrogel to be freeze-dried; and (d) control of the thermal processing immediately prior to freeze-drying.

Pore-forming agents for introducing pores into cross-linked hydrogels can include particles that are insoluble, or only partially soluble, in the solvent used for crosslinking. In one specific embodiment, calcium alginate microbeads added to hydrogels during cross-linking can be used to form pores of a narrowly defined size range. During crosslinking, the insoluble calcium alginate beads become entrapped within the hydrogel. Washing the crosslinked hydrogel in a solution containing sodium citrate converts the calcium alginate to its soluble sodium alginate form which can then be leached from the hydrogel to leave voids corresponding roughly to the size of the entrapped beads. Any other bead-like material capable of being incorporated into the hydrogel matrix during cross-linking and later removed to leave open pores could be used according to the invention. Accordingly, in some embodiments, the invention encompasses the use of microbeads that are insoluble during a crosslinking process but are solulizable in a separate process step.

In another embodiment, cross-linked hydrogels can be formed using oxidized dextrans. When such compositions are vigorously mixed with up to approximately 2 volumes of air, the resultant solid material exhibits a closed pore structure. Subsequent processing of these materials by cycling the material from high to low pressures (i.e. from less than 50 mm Hg to 760 mm Hg) converts this closed pore foam to a structure containing interconnected pores.

In still another embodiment, the addition of water to hydrogels during cross-linking reduces the solids content of the cross-linked hydrogel. These diluted hydrogels, when freeze-dried, form an insoluble scaffold with interconnected pores. Further by manipulating the temperature profile of the hydrogel during the initial freezing cycle, the size of the formed ice crystals can be controlled. A rapid thermal quench from ambient temperatures to temperatures well below the materials eutectic point results in an amorphous solid with relatively small pores. Conversely, a much slower cooling rate promotes the growth of larger ice crystals to provide larger pores.

Dry gel density provides an indirect measure of scaffold density. Such dry gel density can be calculated as the ratio of the mass of a freeze-dried block of a cross-linked hydrogel matrix to its volume calculated from its dimensions measured using digital calipers.

The pore volume of cross-linked gel scaffolds can be estimated from the mass of an organic solvent trapped in the pores of dry gels. As noted above, the dimensions of dry gels can be measured using digital calipers. With knowledge of the mass of the dry hydrogel matrix particles, the dry gels can be transferred to an anhydrous organic solvent, such as cyclohexane or acetone, and the scaffolds allowed to become fully wetted over a period of time (such as about 10 minutes). The wetted gel pieces are transferred to a syringe, and a gentle vacuum is applied to pull the trapped air out of the gel scaffold. Scaffolds completely filled with organic solvent will sink and are translucent, while scaffolds with residual trapped air will float and appear as opaque white foams.

Completely wetted gels are transferred to a pre-weighed container with the organic solvent, and the mass of the solvent wetted scaffold is recorded. The difference between the solvent wetted gel and the dry gel is calculated to yield the mass of the solvent trapped in the pores. The volume of the solvent in the pores (i.e., the pore volume) is calculated using the known density of the solvent. The pore volume is calculated as a ratio against the volume calculated by direct measurement of the gel block to obtain the percent pore volume, or porosity, of the crosslinked hydrogel matrix. particles

The average pore size of the hydrogel matrix particles can vary depending upon the desired use and is generally in the range of about 10 $\mu$m to about 1000 $\mu$m, about 50 $\mu$m to about 900 $\mu$m, about 100 $\mu$m to about 800 $\mu$m, about 150 $\mu$m to about 700 $\mu$m, or about 200 $\mu$m to about 600 $\mu$m. The average interconnectivity pore size of the hydrogel matrix is preferably less than about 200 $\mu$m, less than about 150 $\mu$m, less than about 100 $\mu$m, or less than about 75 $\mu$m. The hydrogel matrix particles can have an average porosity of at least about 25%, at least about 30%, at least about 35%, at least about 40%, at least about 45%, at least about 50%, at least about 55%, or at least about 60%. In other embodiments, the hydrogel matrix particles can have an average porosity of about 30% to about 90%, about 40% to about 85%, or about 50% to about 80%.

In specific embodiments, such as for scaffolds designed for use as bone void filler, hydrogel matrix particles preferably have a pore size of about 200 $\mu$m to about 600 $\mu$m, an interconnectivity pore size of less than about 100 $\mu$m, and a porosity of at least about 50%. These dimensions are particularly useful to permit the infiltration of osteoblasts to populate the implanted scaffold and promote rapid osteointegration.

The particulate crosslinked hydrogel matrix of the present invention facilitates ease of use of cryopreserved cells. As previously pointed out, cryopreservation is a useful method for storing cells and ensuring an ongoing supply of cells, such as stem cells, that typically have a short life-span or are difficult to maintain in active culture. Cryopreservation, however, is not a simple laboratory task, and careful measures must be undertaken to ensure cells being cryopreserved for later use are actually usable when thawed.

Cells for cryopreservation typically must be in the form of a cell suspension. This presents a problem, though, since many cell lines are adherent or semi-adherent and must be grown on some type of cell support, or substrate. Such cells attach to (or are adhered to) the substrate using cell-secreted proteins that form a tight bridge between the cell and the substrate. Thus, the primary step in preparing most cell types for cryopreservation comprises removing the cells from their substrate. One known method consists of treating the cells with an enzyme, such as a proteinase, capable of de-bonding the cells from the substrate by destroying the cell-surface attachments (*e.g.,* trypsinizing). Another method for cell removal includes mechanically scraping the cells loose from the substrate. Both methods are known to cause considerable cell damage ranging from inactivation or destruction of cell surface proteins to partial or complete destruction of the cellular membrane. Accordingly, many cells being placed in cryopreservation are actually in a "weakened" or less viable condition arising from the preparation procedures generally recognized as necessary in cryopreservation. This disadvantage is magnified by the inherent risks to cell integrity arising from the actual freezing and thawing processes themselves.

Various phenomena which can cause damage to cells are known to exist during cryopreservation, such as solution effects, extracellular ice formation, dehydration, and intracellular ice formation. Solution effects are often caused by concentration of solutes in non-frozen solution during freezing of cells as solutes are excluded from the crystal structure of the formed ice, and high salt concentrations can be very damaging to cells. If cells are cooled too slowly, water can migrate out of the cells, which can result in formation of ice in the extracellular space. Too much extracellular ice can cause mechanical damage due to crushing. The migration of water causing extracellular ice formation can also cause cellular dehydration. The cellular stresses associated with dehydration can themselves lead directly to cellular damage. Intracellular ice formation is particularly troubling because, although some organisms and tissues can tolerate some extracellular ice, any appreciable intracellular ice is almost always fatal to cells.

In light of the above, it is desirable to avoid unnecessarily damaging cells prior to subjecting the cells to the inherently harmful conditions associated with cryopreservation. In other words, as the cryopreservation environment itself is so capable of damaging cells, it is desirable to ensure the cells being cryopreserved are in their best possible condition at the time of cryopreservation.

It is clear that cells that have been cryopreserved using known techniques are not in an ideal state at the time of removal from cryopreservation. Accordingly, resuscitation of adherent and semi-adherent cell lines typically requires re-culturing of cells immediately upon thawing. The resuscitated cells are thus provided a recovery time to overcome damage inflicted during removal from culture prior to cryopreservation. This recovery time allows for re-growth of cell surface proteins damaged or removed by the prior enzymatic treatment or to re-establish cell membrane integrity where more widespread damage has occurred. Without this recovery phase, many cryopreserved cells would not be usable *in vivo.* Thus, present cell cryopreservation methods present many difficulties that must be overcome through repetitive lab techniques that are time consuming and costly. However, through use of the bioactive hydrogel matrix of the present invention, it is possible to provide cryopreservation techniques that dispense with several intermediate steps and allow for a seamless transition from active cell culture, to cryopreserved cells, to active cells for *in vivo* use.

Accordingly, in one embodiment, the present invention provides methods for cryopreserving cells. The inventive methods make use of a cross-linked bioactive hydrogel matrix as described herein in particulate form. The particulate hydrogel matrix is both diverse in use and convenient. For example, the hydrogel matrix can be prepared to be in particulate form and provided in commercial quantities in a "ready-to-use" form. The hydrogel matrix can likewise be prepared in the laboratory and made into particulate form at the time of use.

In certain embodiments, the method of the invention comprises combining cells for cryopreservation with the inventive cross-linked bioactive hydrogel matrix in particulate form and subjecting the combined cells and cross-linked bioactive hydrogel matrix to cryopreservation conditions. As used herein "cryopreservation conditions" refers to any set of conditions typically recognized as useful in the art for cryopreserving cells. Accordingly, cryopreservation conditions can simply refer to an environment providing a "cryopreservation temperature", or a temperature sufficiently below zero degrees Centigrade (below 273 K) to slow or stop biological activity within a cell, including but not limited to biochemical reactions within the cell that would lead to cell death. In specific embodiments, a cryopreservation temperature comprises a temperature of less than about -20 °C (253 K), less than about -50 °C (223 K), less than about -80 °C (193 K), less than about -100 °C (173 K), or less than about -130 °C (143 K). As used herein, the term "cryopreserved state" means a state of being at a cryopreserved temperature.

Any freezing apparatus capable of providing prolonged sub-zero temperatures to maintain a cryopreserved state can be used according to the present invention. When liquid nitrogen is used, the cryopreservation temperature typically approaches the boiling point of nitrogen, or about -196 °C (77 K). Freezing and storage may be carried out in the same apparatus, or a first freezing apparatus may be used prior to transfer of frozen samples to a long-term storage apparatus. Liquid nitrogen storage vessels are typically used. Passive freezing methods involving more sophisticated cooling devices, such as the programmable, rate controlled Planer Series Two freezer (available from Planer Products) are now commonly used, particularly in large-scale cryopreservation operations. The NALGENE® Mr. Frosty (available from Nalgene Nunc International) is an example of another freezing apparatus commonly used in the industry. Such freezers typically cool a sample to a temperature of around -80 °C (193 K). Any such methods and apparatuses may be used according to the present invention.

Cryopreservation conditions can further refer to the use of additives, or physical conditions, for promoting vitrification, which decreases damage due to ice crystal formation. For example, cryopreservation conditions according to the invention can comprise adding to the cells a cryoprotectant. Preferably, the cryoprotectant is effective for lowering the freezing temperature of the cell suspension, increasing the viscosity of the cell suspension, or both. Vitrification is generally understood to refer to hardening (to a glass-like state) without formation of ice crystals. Vitrification is promoted by rapid cooling, and can be achieved without cryoprotectants by an extremely rapid drop in temperature (typically megakelvins per second).

Any cryoprotectant recognized as useful in the art for cryopreservation of cells can also be used according to the present invention. Preferably, the cryoprotectant comprises a solute capable of penetrating the cell membrane of the cells for cryoprotection in order to achieve increased viscosity and depressed freezing temperature inside the cell. One of the first compounds found useful as a cryoprotectant was glycerol. Dimethyl Sulfoxide (DMSO) is also recognized as a useful cryoprotectant. DMSO passes through cell membranes more readily than glycerol, but it can be more toxic at higher temperatures. Nevertheless, the most commonly used cryoprotectant today is DMSO, such as at a final concentration of 5% or 10%, however, this is not appropriate for all cell lines *e.g.* HL60 where DMSO is used to induce differentiation. In such cases an alternative such as glycerol can be used (reference can be made to the European Collection of Cell Cultures (ECACC) data sheet for details regarding the types of cryoprotectants that can be used with specific cell lines). Ready-made cell freezing media are widely available (*e.g.,* a combination of DMSO, glycerol, and a serum-free formulation containing DMSO is available from Sigma). Other cryoprotectants, such as methoxylated compounds, are also used in cryoprotection and are often considered less toxic and more penetrating. Still further examples of cryoprotectants include ethanol, ethylene glycol, 2-methoxy ethanol, 1,2-dimethoxyethane, propylene glycol, 1-methoxyl-2-propanol, and glycerol derivatives, such as 3-methoxy-1,2-propanediol or 1,3-dimethoxy-2-propanol.

Various mixtures of such known cryoprotectants alone, or in combination with further compounds, can also be used in cryoprotection methods of the invention. For example, U.S. Patent No. 6,395,467 describes cryoprotectants formed of DMSO, an amide (such as formamide, urea, acetamide, hydroxyurea, or N-methyl formamide), and ethylene glycol or ethylene glycol in combination with propylene glycol. U.S. Patent Application Publication No. 2002/0042131 describes cryoprotectants prepared without the use of DMSO or formamide. Such cryoprotectant formulations are generally based on diols, such as propane-1,2-diol, and can further include compounds, such as sodium chloride, potassium chloride, potassium phosphate monobasic, potassium phosphate dibasic, sodium bicarbonate, and glucose.

The concentration of penetrating cryoprotectants (such as DMSO), and hence the toxicity of the cryoprotectant cocktail, can be reduced by use of non-penetrating cryoprotectants, such as large molecular weight polymers (*e.g.,* polyvinylpyrrolidone or polyethylene glycol) or sucrose. Non-penetrating cryoprotectants are formed of compounds too large to diffuse into cells, but able to assist with vitrification of water (and inhibit de-vitrification) in the extracellular space. Less cryoprotectant is needed inside cells than in the extracellular space because of dehydration (which drives water from cells into the extracellular space) and because cells naturally contain proteins that enhance vitrification. Penetrating cryoprotectants can also be referred to as intracellular cryoprotectants and typically have low molecular weights. Non-penetrating cryoprotectants can also be referred to as extracellular cryoprotectants and typically have relatively high molecular weights (greater than or equal to sucrose (342 daltons)). Intracellular cryoprotectants, such as glycerol and DMSO at concentrations from 0.5 to 3 molar, are effective in minimizing cell damage in many slowly frozen biological systems. Extracellular cryoprotective agents such as polyvinylpyrrolidone or hydroxyethyl starch are often more effective at protecting biological systems cooled at rapid rates. Any of the above cryoprotectants could be used according to the present invention.

Vitrification can be assisted by substances other than cryoprotective agents. For example, carrier solutions can reduce the amount of cryoprotectant needed to vitrify. The carrier solution described in Cryobiology, 27(5):492-510 (1990) is a mixture of salts, dextrose, and glutathione, and is based on the so-called RPS-2 solution used for storing rabbit kidneys. A carrier solution can substitute for water, but is typically only used in a 2-5% range. The carrier solution effect is largely colligative - *i.e.,* molecules getting in the way of water molecules which might otherwise form ice. A good carrier solution will be non-toxic, and by reducing the amount of cryoprotectant needed to vitrify will reduce toxicity from the cryoprotectant.

Another source of assistance for vitrification comes from ice blockers. While cryoprotectants slow ice-crystal growth and formation, ice blockers act specifically against the formation of the ice nuclei that are necessary for freezing to begin. Ice crystals can grow along six symmetric axes: the a-axes, all six axes in the same plane; or the c-axis, which is perpendicular to the plane of the six a-axes. Ice crystal growth at higher temperatures typically occurs along the a-axes, which accounts for the familiar hexagonal shape of snowflakes. Ice blockers can act by three mechanisms: (1) bind to and inactivate heterogeneous nucleating substances, (2) block a-axis growth or, (3) block c-axis growth. Proteins can be useful as ice blockers, and such "anti-freeze proteins" often rely on amino acids, such as threonine and serine to hydrogen-bond with ice to prevent crystal formation (Cryobiology, 41(4):257-279 (2000)). The benefits from ice-blockers can be very great. Toxicity increases exponentially as the cryoprotectant concentrations reach the high levels needed to vitrify. While many cryoprotectants become too viscous to perfuse well at high concentrations, ice blockers typically add little to overall viscosity. Thus, the combination of an ice-blocker with a cryoprotectant can produce a solution that can both perfuse and vitrify.

Combining the cells for cryopreservation with the particulate cross-linked bioactive hydrogel matrix can encompass any method recognized in the art for causing cells to contact a substrate, particularly a substrate in particulate form. Preferably, the cells are combined with the particulate hydrogel matrix in a manner that allows the cells to be retained by the particulate hydrogel matrix. As previously noted, a thermoreversible form of the hydrogel matrix of the present invention has previously been shown useful as a cell culture medium and a composition for preserving cell viability. The particulate crosslinked hydrogel matrix of the invention likewise provides a highly suitable substrate for cell attachment and, upon combination of cells with the particulate hydrogel matrix, the cells will readily adhere to, attach to, or be otherwise retained on at least a portion of the surface of the particles. Thus, the relationship between the cells and the particles can be described such that the cells are at least partially retained on, adhered to, or attached to an exposed surface of the particles. Moreover, as the particulate hydrogel matrix of the invention can be formed to have a specific porosity, it is also possible for cells to adhere to, attach to, or become entrapped within the pores of the particles. Thus, the relationship between the cells and the particles can be described such that the cells are at least partially retained, attached, or adhered within one or more pores present in the particles. By these methods, cells are effectively retained by the particles or carried by the particles. In some embodiments, the cells can be described as being carried by the hydrogel matrix or retained by the hydrogel matrix.

The particulate hydrogel matrix provides a support surface to which cells can attach and carry out normal cell activities, including growth and division, production of cell products (*e.g.,* proteins) and metabolism by-products, and other normal cell activities. Moreover, as described above, the particles can be made to have a particular size and porosity to allow for attachment of a defined number of cells per particle. Accordingly, it becomes possible to easily count and transfer cells based upon the mass or volume of particulate hydrogel matrix and knowing the average number of cells per particle and the average mass or volume per particle.

To combine cells with the hydrogel matrix particles, existing cell cultures can be trypsinized to provide a cell suspension in which the hydrogel matrix fragments are incubated. The cells attach to the fragments and are cultured to allow recovery and to build associations with the material and each other. Thus, when subjected to cryopreservation conditions, the cells have had opportunity to recover and exhibit greater viability in comparison to cells that are cryopreserved immediately after removal from culture. In this manner, the hydrogel matrix fragments perform the function of a cell substrate that can carry through into cryopreservation, and even after cryopreservation can be directly implanted with the cells in therapeutic methods. In this manner, as further described below, the matrix fragments can serve a bioreactor function to allow cell population expansion in suitable culture medium and conditions. In fact, studies have indicated that such seeded fragments that were cultured for a week prior to cryopreservation exhibited better differentiation activity post-thawing than did cells cultured only 1-day prior to freezing.

The cells and the particulate hydrogel matrix can be physically combined according to a number of methods. In specific embodiments, the cells for cryopreservation are present in a cell suspension prior to combination with the particulate hydrogel matrix. This could be achieved by providing the cells for cryopreservation in a cell suspension and contacting the hydrogel matrix particles with the cell suspension. Such passive loading methods simply place the cells and the hydrogel matrix particles in a combination where the natural attractive forces of the cells for an attachment surface leads to loading of the cells on the scaffolds and scaffold fragments provided by the hydrogel matrix particles. In further embodiments, it is possible to use gravitational forces (e.g., centrifugation) or fluid flow (e.g., vacuum loading) to drive or pull cells into a construct. While such active loading methods can be used, they are generally not required in light of the avid binding of cells to the scaffolds provided by the hydrogel matrix. Rather, the hydrogel matrix fragments can simply be incubated with a cell suspension with occasional mixing while being held at incubator temperature.

In one embodiment, the step of combining the cells with the particulate hydrogel matrix can comprise providing the cells for cryopreservation in a cell suspension and adding the particulate cross-linked hydrogel matrix to the cell suspension with mixing. Mixing can be carried out by any conventional means capable of providing sufficient flow within the suspension to cause the cells to come into contact with the particulate hydrogel matrix. Preferably, shearing is limited to avoid damage to cells.

At least a portion of hydrogel matrix particles can rehydrate once added to the suspension. The hydrogel matrix particles, though, are not soluble, and they remain as small fragments of the crosslinked bioactive matrix. This provides further advantages as the hydrogel particles are useful to grow mass quantities of cells in a bioreactor setting. As such, the hydrogel matrix fragments can be seeded with cells in suspension culture and allowed to fill with cells, thus expanding the culture mass or sampling the medium for any product of interest that may be produced and secreted. This aspect of the invention is further described below.

It is also possible to combine the cells and the particulate hydrogel matrix by applying a cell suspension to the particles. For example, an amount of particles can be provided in a container, and the cell suspension can be applied to the particles by conventional methods, such as pouring, drizzling, wiping, or any other method useful for ensuring contact of the cells in the suspension with the hydrogel matrix particles.

A particular advantage of the present invention is the ability to actually use the particulate hydrogel matrix as a substrate for cell attachment. Thus, it is possible, according to certain embodiments, to use the particulate hydrogel matrix as the predominant cell substrate for cell growth. In such embodiments, the present invention encompasses cryopreservation methods where the step of combining the cells and the particulate hydrogel matrix is not required. Rather, the method can simply comprise subjecting to cryopreservation conditions a cross-linked bioactive hydrogel matrix as described herein in particulate form having cells for cryopreservation adhered thereto.

The beneficial aspects of the present invention are even further realized in relation to the resuscitation of cells that have been cryopreserved according to the methods of the invention. As the cells adhered to the hydrogel matrix particles have been able to recover from any damage produced through contact with proteinases or damaged by mechanical means, such as scraping away from a substrate, the cells are in a much "healthier" condition upon being cryopreserved. This translates into improved physical characteristics and improved cell integrity upon thawing. Accordingly, the severity of many of the phenomena described above associated with cell damage during cryopreservation can be lessened simply from the improved cell integrity immediately prior to cryopreservation. Moreover, the physical attachment of the cells to the particulate hydrogel matrix can actually function to stabilize the cells during freezing and still further limit cell damage during the cryopreservation. Accordingly, it is clear that the methods of the present invention are highly beneficial for improving cryopreservation generally by increasing ease of transfer from substrate to cryopreservation medium, as well as increasing the actual viability of the cryopreserved cells.

The advantages of the present invention are further seen, though, in the greatly improved ease of transition between the frozen state and an active form ready for *in vivo* application. Resuscitating cells from a cryopreserved state typically encompasses thawing the cells and then re-culturing the cells to allow for cell recovery. As pointed out previously, this cell recovery time is typically needed to allow the cells to regain viability in light of the procedures used in preparation for cryopreservation.

Specifically, before cryopreserved cells can be used *in vivo,* they typically must be cultured in conditions to allow for regeneration of cell components (such as cell surface proteins) that may have been disabled or destroyed by the proteinase treatment used to remove the cells from culture prior to cryopreservation. Moreover, as the mechanical scraping often used to dislodge cells from culture prior to cryopreservation can disrupt cell membrane integrity, the recovery time is often required to allow for membrane regeneration generally. This recovery period also allows time to increase overall cell count to overcome cell losses due to the methods used in preparing for cryopreservation, as well as the stresses generally related by suspended cells during cryopreservation, including freezing and thawing.

Re-culturing thawed cells to provide recovery time has heretofore simply been an accepted step in the cryopreservation process. The present invention, however, realizes the lack of necessity of this step, as well as the unnecessary cost associated with manpower to oversee the cultures and supplies to maintain the cultures. Moreover, this added step limits the conditions where cryopreserved cells can be used.

According to known methods, use of cryopreserved cells is limited to settings having sufficient laboratory space and materials to carry out culturing of the previously frozen cells. Alternately, use of the cryopreserved cells requires outsourcing of the re-culturing and then purchase of the resuscitated cells. This presents even further unnecessary limitations in that the resuscitated cells must be ordered in advance with the foreknowledge that they must be used immediately upon receipt. All of these inconveniences and undue costs are overcome by the present invention, however.

Cells cryopreserved in combination with the hydrogel matrix particles of the present invention can be used *in vivo* with no need to carry out an intervening culturing step. As described above, cryopreservation of the cells using the hydrogel matrix particles as a suspendable substrate eliminates the need for proteinase treatment, mechanical scraping, or other similar activities required to prepare adherent cells for cryopreservation according to known methods. By doing away with these treatments, the cryopreserved cells retain their cell surface proteins, maintain intact membranes, and generally enter cryopreservation in a healthier, more robust condition. Accordingly, upon thawing, the cells are in a viable condition for *in vivo* use, and re-culturing becomes superfluous.

In an even further advantage, if one desired to re-culture the thawed cells attached to the hydrogel matrix particles, the ability to re-culture is not hindered and is actually facilitated by the present invention. Since the hydrogel matrix of the invention is actually a highly suitable cell culture scaffold, one wishing to re-culture thawed cells that had been cryopreserved according to the invention, such re-culturing could be carried out by simply suspending the cells attached to the hydrogel matrix particles in a suitable culture fluid (*i.e.,* a fluid containing nutrients suitable for the cell types attached to the hydrogel matrix particles). In this manner, it would even be possible to obtain a certain count of cells cryopreserved attached to the hydrogel matrix particles and then expand the cell line after thawing and prior to use. The ability to use the hydrogel matrix as a cell line expanding tool is more fully described below.

Ease of use of cryopreserved cells according to the present invention is further achieved because of the inherent bioactive nature of the particulate hydrogel matrix. As described above, the hydrogel matrix of the invention is fully compatible for use with any living tissues and can particularly be used throughout the human body. The hydrogel matrix has also been shown to avoid immune response and is completely biocompatible.

Even more advantageous, however, is that the hydrogel matrix actually has inherent bioactivity such that it can have a synergistic effect with the cells adhered thereto. Accordingly, it is possible according to the invention to actually improve the effective use of cells *in vivo* by administering the cells adhered to the particulate hydrogel matrix.

The present invention is thus further beneficial in that cryopreserved cells that are combined with the hydrogel matrix particles can be directly used *in vivo* immediately after thawing. Of course, if cryopreservation was previously carried out using a cryoprotectant, such as DMSO, that could be potentially harmful to the cells or undesirable for administration to the human body, thawing could be followed by the requisite steps for isolating the combined cells and hydrogel matrix particles from the cryoprotectant and washing, as desired.

There is no need to remove the cells from the particles because the hydrogel matrix particles are biocompatible. In fact, it can even be desirable to use the cells adhered to the hydrogel matrix particles because of the inherent bioactivity of the hydrogel matrix and the beneficial effects on the cells when combined with the hydrogel matrix. Moreover, there is no concern about the particles persisting for an undesirable period *in vivo,* as the hydrogel matrix can be broken down by bodily process yet remains for a sufficient time to provide cell support. The amount of time that the hydrogel matrix persists can be varied as desired for specific applications.

After thawing (and any optional rinsing steps to remove cryoprotectant), the combined cells and hydrogel matrix particles can be re-suspended in a fluid or other medium suitable for the intended use. For example, the combined cells and hydrogel matrix particles can be re-suspended in any osmotically supportive solution. Preferably, an osmotically supportive solution exhibits an osmolality of about 290-350 mOsm/kg. In certain embodiments, the combined cells and hydrogel matrix particles can be re-suspended in a physiologically compatible buffer, such as the buffer solutions otherwise described herein. Preferably, any physiologically compatible material providing a composition for convenient delivery *in vivo* can be used to re-suspend the combined cells and hydrogel matrix particles. In certain embodiments, the suspension can be substantially flowable, and delivery can be via injection using a syringe or other similar device. In other embodiments, the combined cells and hydrogel matrix particles could be incorporated into a solid or semi-solid composition that could be poured, packed, or otherwise placed into a void or cavity.

In a specific embodiment, the combined cells and hydrogel matrix particles can be re-suspended using the non-cross-linked form of the hydrogel matrix of the invention. As noted above, in an uncrosslinked state, the hydrogel matrix can be provided in a thermoreversible form that is a gel at ambient conditions but is molten under physiologic conditions. Thus, the thermoreversible hydrogel matrix makes a useful medium in that it not only provides for suspension of the cells but further provides a nurturing environment for the cells once placed *in vivo.*

In addition to the above methods of use, the present invention further provides, in multiple embodiments, various compositions of cells combined with the hydrogel matrix particles of the invention. For example, in certain embodiments, the present invention provides a cell-seeded composition.

A cell-seeded composition of the invention can comprise particles of a cross-linked bioactive hydrogel matrix, as described herein, having cells retained by the particles. As previously described, the surface area of a hydrogel matrix particle can vary depending upon the average size of the particles, as well as the porosity of the particles. Thus, in certain embodiments, the cell-seeded composition can comprise hydrogel matrix particles having cells contained within one or more pores formed in the hydrogel matrix particles. Cells being contained within the pores can particularly mean cells that are adhered to the surface of the particle, at least a portion of that surface area being located within a pore formed in the particle. In some embodiments, the invention particularly comprises cell-seeded compositions comprising cells attached to a particulate hydrogel matrix, as described herein, the particles having a specific porosity, as described herein.

The cells adhered to the surface of the hydrogel matrix particles or contained within a pore of the hydrogel matrix particles can be in an active state. As used herein, the term "active state" refers to a condition wherein a cell is able to carry out normal cell processes including, but not limited to growth, nutritional uptake, metabolism, production of proteins, and cell division.

The present invention further contemplates combinations of cells and the hydrogel matrix particles that are in a cryopreserved state. As noted above, the hydrogel matrix particles of the invention are a particularly useful cell attachment substrate that allows for direct cryopreservation of cells while adhered to a substrate. Thus, in one embodiment, the invention provides a cell-seeded composition comprising cells combined with hydrogel matrix particles as described herein, the combination being in a cryopreserved state. Compositions according to this aspect of the invention can also incorporate various further components. For example, in one embodiment, the invention comprises a composition comprising cells, hydrogel matrix particles as described herein, and a cryoprotectant.

As the combined cells and hydrogel matrix particles allow for direct *in vivo* application after thawing, the invention also encompasses combinations that have previously been cryopreserved. For example, in one embodiment, the invention comprises a cell-seeded composition comprising cells combined with hydrogel matrix particles as described herein, wherein the combined cells and hydrogel matrix particles have previously been cryopreserved.

In further embodiments, the invention encompasses combinations of cells and hydrogel matrix particles (whether previously cryopreserved or not) in combination with further components useful for preparing the combination for direct *in vivo* use. For example, in one embodiment, the invention comprises a composition comprising cells, hydrogel matrix particles as described herein, and a suspension medium. In a specific embodiment, the suspension medium comprises a non-crosslinked, thermoreversible hydrogel matrix as described herein.

The methods and compositions of the invention can be used with a large variety of cell types. The invention is particularly useful in relation to cells that are adherent or semi-adherent in nature. Adherent cell types are typically understood to be cells that are unable to grow in free suspension but rather must be adhered to a substrate or surface for proper growth to occur. Semi-adherent cell types grow as a mixed population wherein some cells are adhered to a substrate but some cells are in suspension.

Specific examples of cell types that beneficially undergo cryopreservation according to the methods of the present invention include stem cells (including embryonic stem cells, cord blood stem cells, and adult stem cells), progenitor cells, and other, similar cells that are pluripotent (capable of differentiating into any fetal or adult cell type) or multipotent (capable of differentiating into a limited number of cell types) in nature. Such cells, *ex vivo,* typically must be adhered to a substrate to ensure proper growth and differentiation. Moreover, such cells often tend to be more fragile during culturing than other, more highly differentiated cell types. Accordingly, stem cells, progenitor cells, mesenchymal cells, and similar cells particularly benefit from the present invention since the cells can be adhered to a substrate (the particulate hydrogel matrix) that allows for direct cryopreservation without disturbing the cells by proteinase or mechanical removal means.

Specific types of cells that could be used according to the invention include (without limitation) mesenchymal stem cells, neural stem cells, muscle stem cells, adipose-derived adult stem (ADAS) cells, liver cells, pancreatic cells, chondrocytes, osteoblasts, adipocytes, and fibroblasts (with or without genetic alterations). Of course, other cells could also be used according to the invention.

The present invention is also useful in various methods of treatment. Cellular therapy, or cell therapy, can generally encompass transplantation of human or animal cells to replace or repair damaged tissue and/or cells. Cell therapy has been used to rebuild damaged cartilage in joints, repair spinal cord injuries, strengthen a weakened immune system, treat autoimmune diseases such as AIDS, and help patients with neurological disorders such as Alzheimer's disease, Parkinson's disease, and epilepsy. Further uses have included treatment of a wide range of chronic conditions such as arteriosclerosis, congenital defects, and sexual dysfunction.

Cell therapy typically involves the injection of either whole cells or cell extracts that are xenogenic (typically fetal animal cells, *e.g.,* from sheep, cows, pigs, and sharks), allogenic (from another human donor), or autologous (wherein the cells are extracted from and transplanted back into the same patient). Autologous cell therapy has gained the most acceptance and is generally recognized as the most viable type of cell therapy in light of the immune response. Various applications of cell therapy are currently undergoing research, experimentation, and clinical trials, and the United States Food and Drug Administration has approved the use of at least one cellular therapy technique for repairing damaged knee joints. The procedure involves removing healthy chondrocyte cells from the patient, culturing the cells for a period of time (typically three to four weeks), and then transplanting them back into the damaged knee joint of the patient.

Cell types useful in the methods of the invention are generally unrestricted and can specifically encompass any of the cell types discussed herein, including cells that are, in reference to a patient being treated, autologous, allogenic, or xenogenic. In preferred embodiments, the methods of treatment discussed herein use autologous cells.

The present invention is particularly useful in applications where it is useful to store cells for a period of time for use in later cell therapies. This can include storage of a patient's own cells for later transplantation, as well as storage of a generic cell line (for example, an embryonic stem cell line for use in research or therapies). Accordingly, in one aspect, the invention provides a method of administering viable cells to a site in a patient.

In one embodiment, the method of the invention comprises providing cryopreserved, cell-seeded particles comprising cells and cross-linked bioactive hydrogel matrix particles as described herein, thawing the cryopreserved, cell-seeded particles, and administering the cell-seeded particles to the site in the patient. In further embodiments, the therapeutic method can comprise further steps. For example, the method can comprise harvesting cells from a patient and cryopreserving the harvested cells in combination with the hydrogel matrix particles. In further embodiments, the method can comprise, prior to administering, forming a suspension of the cell-seeded particles. In a preferred embodiment, the suspension is formed using the thermoreversible hydrogel matrix described herein.

In certain embodiments, the methods of the invention can include the use of stem cells attached to the hydrogel matrix particles. Specifically, such could be used for the treatment of lesions that require the repair of bone. Moreover, any connective tissue lesion could be treated according to this aspect of the invention.

In further embodiments, the inventive methods can include the use of adipocytes attached to the hydrogel matrix particles. Such methods can specifically include tissue bulking methods, as well as similar cosmetic applications

As described above, the particulate hydrogel matrix is particularly beneficial according to the invention in light of the ability to use the hydrogel matrix in association with cells across the lifetime of the cells. The particulate hydrogel matrix provides a suitable substrate for cell attachment and growth eliminating the need to remove adherent cells for suspension for cryopreservation. The cells adhered to the particulate hydrogel matrix can be directly cryopreserved without further processing. After thawing, the previously cryopreserved cells, still attached to the hydrogel matrix particles, can be directly used *in vivo.* This diverse utility leads to even further uses for the hydrogel matrix particles in relation to cells.

In certain embodiments, the hydrogel matrix can be used as a substrate for cell culture in facilitating cell line expansion, thus forming a virtual bioreactor. For example, fragments of a cross-linked hydrogel matrix can be used as cell culture carriers for population expansion. In specific embodiments, cells can be seeded onto hydrogel matrix fragments, which are then cultured in a suitable apparatus, such as a spinner flask, to keep the medium mixed and the hydrogel fragments in suspension during cell growth. After cell populations have proliferated to fill the scaffold surfaces of the hydrogel matrix fragments, the cultures can be dissociated (such as using trypsin or collagenase) to free the cells for subculture onto more carriers or for use in specific procedures.

EXPERIMENTAL

The present invention is more fully illustrated by the following examples, which are set forth to illustrate the present invention and are not to be construed as limiting thereof. Unless otherwise indicated, all percentages refer to percentages by weight based on the total weight of the bioactive hydrogel matrix.

EXAMPLE 1

Formation of Cross-Linked Hydrogel Matrix

20 g of dextran (MW 500,000 Da) was weighed into a tared beaker containing 180 g phosphate-buffered saline. The dextran was dissolved with constant stirring and 8 g sodium meta-periodate (available from Sigma, product number S1147) was added to the dissolved dextran. The beaker was wrapped in foil to prevent photo-catalyzed side-reactions, and placed in a refrigerator on a stirring plate for 12 hours at 5°C $\pm$ 3°C. The beaker was removed, 50 mL ethylene glycol was added to consume excess periodate, and the quenching reaction was allowed to proceed for 30 minutes at room temperature. The reaction mixture was pH adjusted to 7.5 $\pm$ 0.5 with 0.1 N NaOH. The reaction products were separated using tangential flow filtration (Filtron Mini-Ultrasette Pall Filtration Products, product number OS100C77). The solution mass was reduced by half, and replaced with a 4-fold volume of phosphate buffered saline. The purified product was reduced to a final volume of 100 mL. The final product was filter sterilized as a 20% dextran solution, and stored frozen until use. Hydroxylamine titration showed that this dextran was 20% oxidized.

A vial of a thermoreversible hydrogel matrix comprising gelatin and dextran and a vial of sterile filtered oxidized dextran were held at 39°C for 30 minutes to melt the hydrogel and warm the oxidized dextran. An aliquot of 10 mL hydrogel was added to a 50 mL centrifuge tube, and rapidly mixed with 5 mL of oxidized dextran. The solution was cast into a 100 mm culture plate, and gently swirled to form a uniform film across the bottom of the dish.

The reactive gel was allowed to cross-link at room temperature. The gel was washed with Medium 199, at 37 °C by flooding the surface of the gel with phenol red containing Medium 199. The Medium 199 overlay was replaced as required to maintain a neutral pH.

A tissue biopsy punch was used to produce 8 mm discs of cross-linked gel. Individual discs were placed in a 15 mL centrifuge tube containing 10 mL Medium 199 and were incubated at 37°C for 2 weeks. Cross-linked gels were insoluble at 37°C and retained their initial shape.

EXAMPLE 2

Effect of Dextran Oxidation on Gel Strength

20 g of dextran (MW 500,000 Da) (available from Sigma, St. Louis, MO) was added to a tared beaker containing 200 mL of phosphate buffered saline (PBS) and stirred to form a uniform solution. A further 8 g of sodium meta-periodate was added to the dextran solution, which was wrapped in foil, and allowed to stir overnight at 5°C $\pm$ 3°C. The reaction was quenched with 50 mL ethylene glycol, and the solution was adjusted with 0.1 M NaOH to a pH of 7.5 $\pm$ 0.5. The product was purified using tangential filtration, and concentrated to a 20% dextran solution. Sterile filtered solutions were stored frozen until use. Hydroxylamine titration showed that this dextran was 18% oxidized. Frozen samples showed no loss in oxidation levels after 8 months storage at 20°C $\pm$ 5°C.

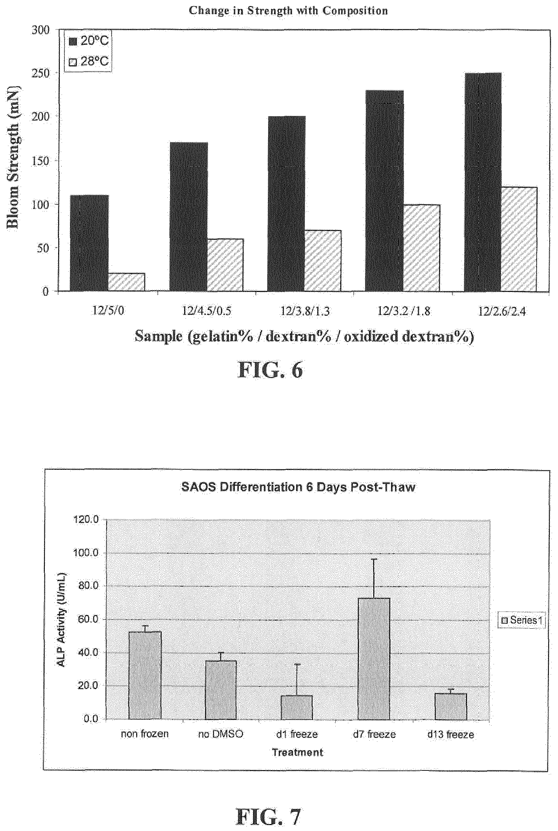

A series of thermoreversible hydrogel and oxidized dextran formulations were prepared with fixed total gelatin concentration (12%) and increasing concentrations of oxidized dextran. As illustrated in FIG. 6, the strength of the cast gels increased as the concentration of oxidized dextran increased. Blends of fixed gelatin concentration and varying oxidized dextran concentration were tested for resistance to compression at two temperatures, 20°C and 28 °C. Gel strength increased with increasing oxidized dextran content and decreasing temperature.

EXAMPLE 3

Use of Hydrogel Matrix as Cell Culture Substrate

20 g of dextran (MW 68,000 Da) (available from Sigma, St. Louis, MO) was added to a tared beaker containing 200 mL of phosphate buffered saline (PBS) and stirred to form a uniform solution. A further 8 g of sodium meta-periodate was added to the dextran solution, which was wrapped in foil, and allowed to stir overnight at 5°C $\pm$ 3°C. The reaction was quenched with 50 mL ethylene glycol, and adjusted with 0.1 M NaOH to a pH of 7.5 $\pm$ 0.5. The product was purified using tangential filtration, and concentrated to a 20% dextran solution. Sterile filtered solutions were stored frozen until use. Hydroxylamine titration showed that this dextran was 14% oxidized.

A thermoreversible hydrogel comprising gelatin and dextran was melted and added to several sets of mixtures of native and oxidized dextrans, mixed and cast into a T-25 culture flask. The concentration of oxidized dextran in each sample ranged from about 3% to about 21%.

The cast gels were allowed to cure at 5°C ($\pm$ 3 °C) overnight, and were washed extensively at 37 °C with phenol red containing Medium 199 (available from Sigma Chemical Company, St. Louis, MO) until no further change in pH was evident colorimetrically. The material was rinsed extensively over four days with culture medium to neutralize residual acidic components.

Flasks containing 12% oxidized dextran were used for further cell culture studies. Normal neonatal human skin fibroblasts were provided in 6 mL of serum-containing culture medium and allowed to interact with the material over an additional two weeks at 37 °C. After 24 hours, cells appeared to maintain normal health, showed attachment to the material by way of cytoplasmic processes, and also exhibited formation of multi-cell clusters. When observed 5 days later, one flask showed large cell aggregates that had formed in the culture and stellate cells in the lower layers of the culture where the large aggregates were attached to the cross-linked hydrogel material. Over the subsequent week, these aggregates continued to grow in size and appeared to contain healthy cells. Cultures were imaged at this time, and the resulting figures (not included herein) showed the appearance of the cell aggregates rising above the material surface with elongated processes and individual cells connecting the structure to the hydrogel substrate.

After approximately one month of exposure to the cross-linked material, cells were successfully dissociated from the hydrogel-containing flasks and replated onto standard tissue culture plastic surfaces, where they were observed to grow readily and showed a morphology similar to that of normally cultured fibroblasts.

EXAMPLE 4

Use of Cross-Linked Hydrogel Matrix as Cell Culture Substrate

A cross-linked hydrogel was prepared according to Example 1 above, wherein the hydrogel was comprised of 12% gelatin and 5% oxidized dextran (MW 500,000 Da). After manufacture, 5 mL of the cross-linked hydrogel was dispensed into a T-25 flask, to which was added 5 mL of culture medium (IMDM containing 10% FBS). The cross-linked hydrogel was solid at incubator temperature (37°C). At 4 hours post-addition, the added medium had undergone a color change from red to light yellow, indicating a change in solution pH toward acidic. The initial 5 mL of culture medium was removed and a second 5 mL quantity was added to test the buffering capacity of the medium. Three days later, the same color change was observed. The culture medium was again removed and replaced with an additional 5 mL of culture medium. One day later, the there was minimal color change indicating neutralization of acidic leachables from the material. On the same day, a population of human skin fibroblasts (product number CCD-1112Sk, American Type Culture Collection) was dissociated and prepared for seeding into the flask. The cells were seeded in fresh medium at a 1:6 dilution in 6 mL total volume, and the flasks were returned to the incubator. After one hour, the cells were extending pseudopodia to connect with the cross-linked hydrogel, but were not yet well attached. After approximately 4 additional hours incubation, no additional attachment was observed. After one more day, approximately 20% of the cells appeared to be forming aggregate-like structures, with aggregates ranging in size from about 2 to about 10 cells and attachment processes extending from the cells. The existing medium was poured off into a new T-25 flask and 3 mL of fresh medium was added to the culture.

The following day, the original and the replated cells were examined. Both cell populations appeared rounded and unhealthy, and the transplanted cells had not attached to the new flask surface.

Five days later (nine days from start of experiment), a third flask was examined, containing human skin fibroblasts, cross-linked hydrogel, and culture medium, which had been incubated undisturbed. This sample exhibited large aggregates. When checked again the following day, the aggregated cell clusters had grown in size and resembled embryo-like structures. Examination six days later revealed large multicellular structures on top of the cross-linked hydrogel with some cells apparently growing into the hydrogel at some sites.

EXAMPLE 5

Cross-Linking of Hydrogel Matrix

1.5 mL of a 0.5 mg/mL solution of Bis-[β-(4-azidosalicylamido)ethyl]disulfide (BASED), a crosslinking agent, in dimethyl sulfoxide (DMSO), is added to a foil-wrapped vessel containing 15 mL of liquid thermoreversible hydrogel containing gelatin and dextran. Photoactivated non-specific cross-linking of the thermoreversible hydrogel occurs upon exposure of the reactive mixture to long-wavelength light, such as that provided by continuous exposure to a 550-watt bulb (flood light used in photography). Longer exposure times demonstrated better cross-linking.

EXAMPLE 6

Preparation of Hydrogel Matrix Particles for use as Cell Culture Substrate

A cross-linked hydrogel matrix was prepared according to EXAMPLE 1 and lyophilized to form solid blocks of the hydrogel matrix. The hydrogel matrix blocks were milled with either two 10-second blender bursts followed by mortar and pestle grinding or blended with more than twenty blender pulses until no further disruption was noted. Ground material was then sieved through a screen with a pore size of 707 microns (i.e., a 25 mesh screen). Particles retained by the screen were poured back into the mortar, ground again and the resulting material returned to the sieve apparatus. The sieved material (having a particle size of less than 707 microns) was poured into a Petri plate, sealed in Parafilm and stored at room temperature in the dark.

EXAMPLE 7

Cell-Seeded Hydrogel Matrix Particles

A 0.02 gm sample of milled hydrogel matrix scaffold from Example 6 was dispensed into two 50 mL tubes followed by 1 mL of McCoy's 5A culture medium (available from Sigma-Aldrich), containing 15% serum. Tubes were placed in an incubator while cultured human SAOS-2 osteoblast cells (passage 40 at 100% confluency) were dissociated. Collected cells were resuspended in 9 mL of medium, and 4 mL was added to each tube. A cell count indicated 3.3 million cells were provided to each sample of milled hydrogel matrix preparation in a total of 5 mL. The tubes were capped and placed at a steep angle in the incubator for 30 minutes with occasional mixing.

A 0.2 mL sample from each tube was dispensed into a 24-well plate, 0.5 mL Live-Dead staining solution was added, and the capped tubes were returned to 37 °C. Additional samples of the seeding scaffolds were taken at 60 and 90 minutes and were also stained. After staining, the wells were examined under combined fluorescent and incandescent illumination, and images of the attached cells on the material were captured. The materials bound cells avidly and were well seeded after the initial 30 minutes incubation. The 60- and 90-minute samples were also well decorated with cells, some of which could be seen extending pseudopodia as they spread out and attached to neighboring surfaces on the fragments. Rocking the plate vigorously on the microscope stage showed no tendency for the cells to detach from the fragments.

The remaining material was aspirated with a 2 mL pipette and transferred to a 12-well plate lacking cell-attachment enhancing surface coating, and placed in the incubator overnight. After overnight incubation, the hydrogel matrix fragments were still loaded with cells. Samples from each preparation were cryopreserved, leaving some in culture to allow better cell covering of the surfaces.

EXAMPLE 8

Cryopreservation of Cell-Seeded Hydrogel Matrix Particles

For cryopreservation, 0.5 mL aliquots of the cell-seeded particles from EXAMPLE 7 were aspirated and transferred to Nunc cryotubes. Flakes of crosslinked hydrogel matrix particles still in the wells were easily flushed up into suspension with a pipette tip. For one set of samples, centrifugation was done at 116 times gravity to 'pellet' the particles and allow removal of the supernatant. One mL of SAOS cell cryopreservation medium (McCoy's 5A medium with 15% serum and 5% DMSO) was added, and the tube was gently agitated to mix the slurry and then put on ice. For the other 0.5 mL samples, 0.5 mL of a cryopreservation solution containing 2x DMSO (10%) was added directly, followed by immediate mixing. All samples were incubated 10 minutes on ice and then transferred to conditions of -80 °C in a Styrofoam carrier. The leftover material was transferred to new wells and provided 1 mL of culture medium, thus removing the cell-seeded flakes from any cells coating the well bottoms.

Six days later, some fragments were sampled and stained with the Live-Dead reagents in the following fashion. Two 100 $\mu$L samples were removed from each of the preps in the 12-well plate and transferred to separate wells of a 24-well plate. One-half mL of stain solution was added to each; one well from each preparation got the complete stain, while others were stained with the Dead stain only. No color was visible in the preparations treated with the Dead reagent alone. In the L/D stained materials, viable cells were found filling in the outer edges and the inner aspects of the fragments.

These preparations were sampled for cryopreservation at day 7 post-seeding by transferring 0.5 mL of the medium plus carriers to a cryotube and adding 0.5 mL of the cryopreservation fluid with 2x DMSO. The suspension was mixed and placed on ice for 10 minutes after which the vials were transferred to the -80 °C freezer. All such preparations were transferred to a liquid nitrogen cryounit after 18-24 hr at -80 °C.

At 13 days post-seeding, another 100 $\mu$L from each of the wells was transferred to a 24-well plate and stained with 1 mL of the Live-Dead reagent. Examination and imaging of the preps revealed that all fragments were very well covered with cells. Of the 20-30 fragments from each preparation, all contained cells, and all cells were viable.

After more than a month at liquid nitrogen temperatures, samples were thawed for *in vitro* assessments. Vials frozen in at day 1 were sampled first to assess viability and differentiation in conditions where cells were given the least time to attach to the scaffolds - as a worst case situation. Samples were thawed rapidly in a 39 °C water bath, and the contents transferred to a 6-well plate well using a P1000 pipettor. Each tube was washed with an additional one mL of fresh medium at room temperature, and this was added to the plate well. This handling captured all of the cells present, whether attached to the scaffold or not after thawing. Some samples were washed after thawing by adding their contents to 5 mL of warm medium and performing a low-speed spin (600 rpm; 42 x *g*) to collect the cross-linked hydrogel matrix flakes with attached cells. As much of the supernatant as possible was aspirated, leaving approximately 30 $\mu$L at the bottom. Flakes were then collected by bringing the tube contents up to 2 mL with fresh medium, resuspending with mixing, and transferring to a 6-well plate well. The volume of medium used provided a thin layer of fluid to encourage cell interaction with the well surface. These scaffold preparations were followed in culture at 37 °C to assess whether cells were present and able to migrate onto the well surfaces.

At 1 day post-thaw the unwashed preparation showed many cells associated with the material. There were also many apparently viable cells attached to the well surface. The washed fragments were composed mostly of the dense granular material with some cells discernable around the edges. Each preparation was sampled for Live/Dead staining, which revealed that all flakes were well loaded with viable cells. Cell density was similar to that seen prior to freezing. The washed material was cleaner; all single separated cells and other debris were removed by the rinse and only flakes filled with viable cells were present. These results showed that even after only a brief *in vitro* loading period [~18 hr] SAOS cells remained attached and viable throughout the cryopreservation process.

When examined three days post-thaw, the non-washed preparation showed great looking cross-linked hydrogel matrix fragments along with debris. The debris was mostly rounded-up cells and cell fragments; there were also some cells attached and spread out on the well bottom. The washed preparation also had great looking cross-linked hydrogel matrix fragments, many of which had seeded 20-40 cell colonies where they were touching the well bottom (as expected).

The cross-linked hydrogel matrix fragments from either preparation were clearly full of viable, active cells able to grow and seed a tissue culture surface like normal cells would do.

EXAMPLE 9

Examination of Viability and Differentiation in Cultured Cryocarriers Post-Thaw

To examine the differentiation capabilities of cells loaded on hydrogel matrix scaffolds following recovery from liquid nitrogen storage, a study was done in which samples of culture medium were tested for the presence of alkaline phosphatase (ALP) after day 1 and day 7 of culture. A negative control (fragments frozen without adding the DMSO cryoprotectant to the freezing cocktail) was also included. Samples from carriers frozen at days 1, 7, and 13 post-seeding were used.

Viability and cell coverage on the fragments was assessed at culture day 1 with Live/Dead staining. Negative control (without DMSO) fragments were free of cells, except in rare cases where a sparse individual cells were present and viable. Unwashed carriers frozen in after 7 days of culture post-seeding showed many viable cells present on each fragment. All fragments contained cells, although the dense granular areas seemed more sparsely seeded. Washed fragments frozen 7 days after seeding showed well-seeded fragments with all cells viable. It was also noted in the preparations that had not been rinsed prior to plating there was no tendency for cells to leave the fragments and attach to the well bottom.

Medium from these carrier cultures was sampled at day 6 for the ALP study. Two hundred $\mu$L of culture medium was sampled at each time point and placed at -80 °C until all samples were collected. Media samples were then thawed and 50 $\mu$L aliquots were tested for ALP activity colorimetrically using the ALKALINE PHOSHATASE LIQUICOLOR® kit (Stanbio, Boerne, TX) and compared with a human serum positive control. FIG. 7 illustrates the levels of ALP activity in units per mL of culture medium over the 6-day culture period.

The results from the ALP study indicate that according to the methods of the present invention, SAOS cells seeded on scaffolds, frozen and stored for more than a month, thawed, and cultured release alkaline phosphatase (a marker for osteogenic differentiation) at levels equivalent to that seen in cultures carried out under routine culture conditions. Comparison of the results from seeded carriers frozen after varying time in vitro for cell recovery and proliferation, suggests that optimal conditions for these cells is one week in culture post-seeding.

Many modifications and other embodiments of the invention will come to mind to one skilled in the art to which this invention pertains having the benefit of the teachings presented in the foregoing descriptions and the associated drawings. Therefore, it is to be understood that the invention is not to be limited to the specific embodiments disclosed herein and that modifications and other embodiments are intended to be included within the scope of the appended claims. Although specific terms are employed herein, they are used in a generic and descriptive sense only and not for purposes of limitation.

**Claims**

1. A bone filler comprising porous particles of a lyophilized bioactive hydrogel matrix comprising a polyglycan cross-linked to a polypeptide, and comprising cryopreserved cells retained on an exposed surface of the porous particles or retained within one or more pores present in the porous particles.

2. The bone filler of claim 1, wherein the porous particles have an average pore size of about 10 $\mu$m to about 1000 $\mu$m.

3. The bone filler of claim 1, wherein the porous particles have an average interconnectivity pore size of less than about 200 $\mu$m.

4. The bone filler of claim 1, wherein the porous particles have an average porosity of at least about 25%.

5. The bone filler of claim 1, wherein the porous particles have an average particle size of about 0.01 mm to about 2 mm.

6. The bone filler of claim 1, wherein the porous particles have an average pore size of about 200 $\mu$m to about 600 $\mu$m, an average interconnectivity pore size of less than about 100 $\mu$m, and an average porosity of at least about 50%.

7. The bone filler of claim 1, wherein the polyglycan comprises dextran, and the polypeptide comprises gelatin.

8. The bone filler of claim 1, wherein the lyophilized bioactive hydrogel matrix further comprises one or more enhancing agents selected from the group consisting of polar amino acids, intact collagen, divalent cation chelators, and combinations thereof.

9. The bone filler of claim 1, wherein the cryopreserved cells include progenitor cells.

10. A bone filler comprising cryopreserved cells for use in cell therapy for administering viable cells to a site in a human or animal, wherein the cells are cryopreserved while retained on an exposed surface or within one or more pores present in particles of a lyophilized bioactive hydrogel matrix comprising dextran cross-linked to gelatin, and wherein the cells are administered to the site.

11. The bone filler and cells for use according to claim 10, wherein the cells include progenitor cells.

12. The bone filler and cells for use according to claim 10, wherein the particles of the lyophilized bioactive hydrogel matrix are defined by one or more of the following:

an average pore size of about 10 $\mu$m to about 1000 $\mu$m, preferably about 200 $\mu$m to about 600 $\mu$m;
an average porosity of at least about 25%, preferably about 30% to about 90%;
an average particle size of about 0.01 mm to about 2 mm;
further comprising one or more enhancing agents selected from the group consisting of polar amino acids, intact collagen, divalent cation chelators, and combinations thereof.

13. The bone filler and cells for use according to claim 10, wherein the cryopreserved cells are thawed prior to being administered to the site.

FIG. 1

FIG. 2a

FIG. 2b

**FIG. 3**

Aggregation of Multiple Cell Types in Hydrogel Matrix

FIG. 4

FIG. 5

Change in Strength with Composition

**FIG. 6**

**FIG. 7**

Europäisches
Patentamt

European
Patent Office

Office européen
des brevets

**EUROPEAN SEARCH REPORT**

Application Number

EP 18 15 2529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/118230 A1 (HILL RONALD S [US] ET AL) 2 June 2005 (2005-06-02)<br>* paragraph [0002] *<br>* paragraph [0015] *<br>* paragraph [0083] - paragraph [0085] *<br>* example 2 * | 1-13 | INV.<br>A01N25/02<br>A61K9/14<br>A61L24/04<br>A01N1/02<br>C08B37/02<br>C08J3/075 |
| X | US 2003/232746 A1 (LAMBERTI FRANCIS V [US] ET AL) 18 December 2003 (2003-12-18)<br>* paragraph [0010] *<br>* paragraph [0032] - paragraph [0033] *<br>* paragraph [0049] *<br>* paragraph [0058] *<br>* paragraph [0067] *<br>* paragraph [0072] *<br>* figure 6 * | 1-13 | C08J3/24<br>C12N5/00 |
| X | CHEN ET AL: "Periodontal regeneration using novel glycidyl methacrylated dextran (Dex-GMA)/gelatin scaffolds containing microspheres loaded with bone morphogenetic proteins",<br>JOURNAL OF CONTROLLED RELEASE, ELSEVIER, AMSTERDAM, NL,<br>vol. 121, no. 1-2,<br>31 July 2007 (2007-07-31), pages 81-90, XP022179909,<br>ISSN: 0168-3659, DOI:<br>10.1016/J.JCONREL.2007.05.023<br>* the whole document * | 1-13 | |

TECHNICAL FIELDS
SEARCHED    (IPC)

A01N
C12N
A61K
A61L

-/--

The present search report has been drawn up for all claims

| Place of search | Date of completion of the search | Examiner |
|---|---|---|
| Munich | 19 April 2018 | Staber, Brigitte |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 2

Europäisches
Patentamt

European
Patent Office

Office européen
des brevets

**EUROPEAN SEARCH REPORT**

Application Number

EP 18 15 2529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MALAFAYA ET AL: "Natural-origin polymers as carriers and scaffolds for biomolecules and cell delivery in tissue engineering applications", ADVANCED DRUG DELIVERY REVIEWS, ELSEVIER, AMSTERDAM, NL, vol. 59, no. 4-5, 9 June 2007 (2007-06-09), pages 207-233, XP022110905, ISSN: 0169-409X, DOI: 10.1016/J.ADDR.2007.03.012 * table 10 * ----- | 1-13 | |
| Y | US 2001/051834 A1 (FRONDOZA CARMELITA G [US] ET AL) 13 December 2001 (2001-12-13) * paragraph [0017] - paragraph [0018] * * paragraph [0021] * * example 2 * ----- | 1-13 | |

TECHNICAL FIELDS
SEARCHED (IPC)

The present search report has been drawn up for all claims

| Place of search | Date of completion of the search | Examiner |
|---|---|---|
| Munich | 19 April 2018 | Staber, Brigitte |

EPO FORM 1503 03.82 (P04C01)

page 2 of 2

## ANNEX TO THE EUROPEAN SEARCH REPORT
## ON EUROPEAN PATENT APPLICATION NO.

EP 18 15 2529

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2018

| Patent document cited in search report | | | Publication date | Patent family member(s) | | | Publication date |
|---|---|---|---|---|---|---|---|
| US 2005118230 | A1 | | 02-06-2005 | AU | 2004285480 | A1 | 12-05-2005 |
| | | | | CA | 2543255 | A1 | 12-05-2005 |
| | | | | EP | 1677846 | A2 | 12-07-2006 |
| | | | | EP | 2514445 | A2 | 24-10-2012 |
| | | | | ES | 2522575 | T3 | 17-11-2014 |
| | | | | NZ | 547140 | A | 25-09-2009 |
| | | | | NZ | 581804 | A | 28-10-2011 |
| | | | | US | 2005118230 | A1 | 02-06-2005 |
| | | | | US | 2008145404 | A1 | 19-06-2008 |
| | | | | US | 2009123547 | A1 | 14-05-2009 |
| | | | | US | 2009124552 | A1 | 14-05-2009 |
| | | | | US | 2012321597 | A1 | 20-12-2012 |
| | | | | US | 2012328700 | A1 | 27-12-2012 |
| | | | | WO | 2005042048 | A2 | 12-05-2005 |
| US 2003232746 | A1 | | 18-12-2003 | AT | 410198 | T | 15-10-2008 |
| | | | | AT | 420672 | T | 15-01-2009 |
| | | | | AU | 2003213253 | A1 | 09-09-2003 |
| | | | | AU | 2003215330 | A1 | 09-09-2003 |
| | | | | CA | 2476653 | A1 | 04-09-2003 |
| | | | | CA | 2476656 | A1 | 04-09-2003 |
| | | | | EP | 1476202 | A1 | 17-11-2004 |
| | | | | EP | 1476204 | A1 | 17-11-2004 |
| | | | | ES | 2314187 | T3 | 16-03-2009 |
| | | | | ES | 2318115 | T3 | 01-05-2009 |
| | | | | JP | 4489437 | B2 | 23-06-2010 |
| | | | | JP | 4624678 | B2 | 02-02-2011 |
| | | | | JP | 2005524425 | A | 18-08-2005 |
| | | | | JP | 2005528933 | A | 29-09-2005 |
| | | | | NZ | 535136 | A | 31-03-2006 |
| | | | | NZ | 535369 | A | 24-02-2006 |
| | | | | US | 2003232198 | A1 | 18-12-2003 |
| | | | | US | 2003232746 | A1 | 18-12-2003 |
| | | | | US | 2008199508 | A1 | 21-08-2008 |
| | | | | US | 2010226961 | A1 | 09-09-2010 |
| | | | | US | 2012076868 | A1 | 29-03-2012 |
| | | | | US | 2013189371 | A1 | 25-07-2013 |
| | | | | US | 2014066694 | A1 | 06-03-2014 |
| | | | | US | 2015140116 | A1 | 21-05-2015 |
| | | | | US | 2017182215 | A1 | 29-06-2017 |
| | | | | WO | 03072155 | A1 | 04-09-2003 |
| | | | | WO | 03072157 | A1 | 04-09-2003 |
| US 2001051834 | A1 | | 13-12-2001 | US | 2001051834 | A1 | 13-12-2001 |
| | | | | US | 2004117033 | A1 | 17-06-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6231881 B
- US 6730315 B
- US 6261587 B
- US 6352707 B
- US 6264992 B
- US 4829000 A
- US 6284284 B

- US 6303765 B
- US 20030232746 A
- US 6011008 A
- US 20050118230 A
- US 6395467 B
- US 20020042131 A

**Non-patent literature cited in the description**

- **HYNES, S.O.** Integrins: a family of cell surface receptors. *Cell,* 1987, vol. 48, 549-554
- **SCHACHT, E.** Hydrogels prepared by crosslinking of gelatin with dextran dialdehyde. *Reactive & Functional Polymers,* 1997, vol. 33, 109-116
- **E. H. A. DE HOOG ; R. H. TROMP.** *Colloids and Surfaces A: Physicochemical and Engineering Aspects,* vol. 213 (2-3), 221-234
- **DEAN et al.** Affinity Chromatography: A Practical Approach. IRL Press
- **KAWAI, K. et al.** *Biomaterials,* 2000, vol. 21, 489-499

- **WISSINK, M.J.B. et al.** *Biomaterials,* 2001, vol. 22, 2291-2299
- **BRASS, L.F. ; BENSUSAN, H.** *The Journal of Clinical Investigation,* 1974, vol. 54, 1480-1487
- **JAFFE, R. ; DYKIN, D.** *Journal of Clinical Investigation,* 1974, vol. 53, 875-883
- **POSTLEWAITHE, A.E. ; KANG, A.H.** *The Journal of Experimental Medicine,* 1976, vol. 143, 1299-1307
- **REDDI, A.H.** Biochemistry of Collagen. Plenum Press, 1976, 449-477
- *Cryobiology,* 1990, vol. 27 (5), 492-510
- *Cryobiology,* 2000, vol. 41 (4), 257-279